(19) Europäisches Patentamt
European Patent Office
Office européen des brevets

(11) Veröffentlichungsnummer: **0 215 739 B1**

(12) **EUROPÄISCHE PATENTSCHRIFT**

(45) Veröffentlichungstag der Patentschrift: **01.09.93**

(51) Int. Cl.[5]: **C07D 499/00**, C07D 205/08

(21) Anmeldenummer: **86810403.5**

(22) Anmeldetag: **05.09.86**

(54) **Synthese von Beta-Lactamverbindungen.**

(30) Priorität: **11.09.85 CH 3928/85**
**06.03.86 CH 919/86**

(43) Veröffentlichungstag der Anmeldung:
**25.03.87 Patentblatt 87/13**

(45) Bekanntmachung des Hinweises auf die Patenterteilung:
**01.09.93 Patentblatt 93/35**

(84) Benannte Vertragsstaaten:
**AT BE CH DE FR GB IT LI LU NL SE**

(56) Entgegenhaltungen:
**EP-A- 0 058 317**
**EP-A- 0 125 208**

**JOURNAL OF THE AMERICAN CHEMICAL SOCIETY, Band 104, Nr. 22, 1982, Seiten 6138-6139; A. AFONSO et al.: "New synthesis of penems, the oxalimide cyclization reaction"**

**JOURNAL OF THE CHEMICAL SOCIETY, CHEMICAL COMMUNICATIONS, 1971, Seite 570; E. CORRE et al.: "Synthesis of 2-acetyl-3-aryl-1,1-dicyano-2-methyl-cyclopropanes"**

**SYNTHESIS, 1980, Seiten 642-644; H. FOUDUET et al.: "A convenient synthesi of poly substituted cyclopropanes"**

(73) Patentinhaber: **CIBA-GEIGY AG**
**Klybeckstrasse 141**
**CH-4002 Basel(CH)**

(72) Erfinder: **Hungerbühler, Ernst, Dr.**
**Zeisigweg 4**
**CH-4310 Rheinfelden(CH)**
Erfinder: **Kalvoda, Jaroslav, Dr.**
**Leimgrubenweg 21**
**CH-4102 Binningen(CH)**

**EP 0 215 739 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein verbessertes Verfahren zur Herstellung von Verbindungen der Formel

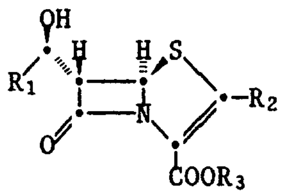

(I),

oder Salzen davon, dadurch gekennzeichnet, dass man

a) eine Verbindung der Formel

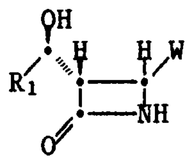

(II)

mit einer den Thiocarbonsäurerest der Formel

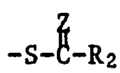

(III)

einführenden Verbindung behandelt,

b) eine erhältliche Verbindung der Formel

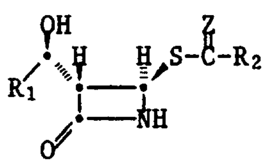

(IV)

mit einem den Acylrest eines Oxalsäurehalbesters der Formel

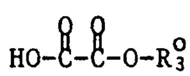

(V)

einführenden Acylierungsmittel behandelt,

c) eine erhältliche Verbindung der Formel

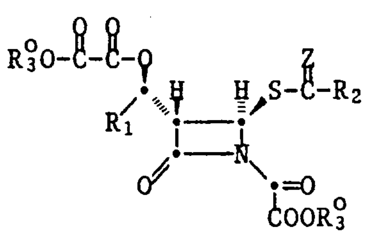

(VII)

mit einer organischen Verbindung des dreiwertigen Phosphors behandelt, und
d) in einer erhältlichen Verbindung der Formel

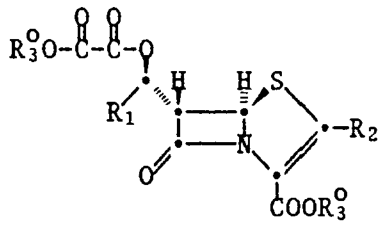

(VIII),

die Oxalylestergruppe -CO-COO-$R_3^o$ und gewünschtenfalls die Schutzgruppe $R_3^o$ und/oder eine gegebenenfalls im Rest $R_2$ vorhandene Schutzgruppe $R_2^o$ in beliebiger Reihenfolge abspaltet und durch Wasserstoff ersetzt, und gewünschtenfalls eine erhältliche Verbindung der Formel I mit salzbildender Gruppe in ein Salz oder ein erhältliches Salz in die freie Verbindung überführt, in welchen Verbindungen $R_1$ Wasserstoff oder Niederalkyl, $R_2$ ein organischer Rest, der eine funktionelle Gruppe tragen kann, die gegebenenfalls durch eine übliche Schutzgruppe $R_2^o$ geschützt sein kann, $R_3$ Wasserstoff oder eine übliche Carboxylschutzgruppe $R_3^o$ , W eine gegen einen Thiocarbonsäurerest der Formel III austauschbare Gruppe und Z Sauerstoff oder Schwefel darstellen, wobei $R_1$, $R_2$ und $R_3$ in der Penenichemie bekünnte Gruppen sind.

Verfahren zur Herstellung von Penemverbindungen und dafür geeigneten Zwischenprodukten sind beispielsweise aus den Europäischen Patentanmeldungen EP-A-0 125 208 und EP-A-0 058 317 bekannt. Bei den bekannten Verfahren lag die 1'-Hydroxygruppe in Verbindungen der Formel II bei Thiolisierung zu den Verbindungen der Formel IV üblicherweise in geschützter Form vor, da angenommen wurde, dass dadurch eine höhere Stereospezifität und eine höhere Ausbeute an trans-Verbindungen der Formel IV erreicht werden würde. Identische Oxalylestergruppen am 1-Stickstoffatom und an der 1'-Hydroxygruppe in Verbindungen der Formel VII, von den die eine als Penembaustein und die andere als Schutzgruppe dient, wurden bisher nicht verwendet. Aus A. Afonso et al, J. Am. Chem. Soc. 1982, 104, 6138-6139, ist die Anlagerung der Allyloxalylgruppe an das 1-N-Atom in Verbindungen vom Typ der Formel IV mit durch Trichlorethoxycarbonyl geschützter Hydroxygruppe bekannt. Solche Verbindungen können mit mindestens zwei Molekülen Triethylphosphit zu Penemen ringgeschlossen werden, wobei das intermediäre entstehende Carben bevorzugt mit der Thioestergruppe reagiert, anstatt mit der Allylgruppe einen Cyclopropanring zu bilden. Der Publikation kann nicht entnommen werden, ob Allyloxalylesterverbindungen der Formel VI analog $\alpha$-Ketoestern mit Triethylphosphit reagieren würden, wobei dann entweder Cyclopropanverbindungen und/oder 1,4-Oxathiacyclohexenverbindungen entstehen könnten.

Die erfindungsgemässe Verbesserung des Verfahrens beruht auf einigen überraschenden Tatsachen. So ist besonders überraschend, dass die Verwendung der Verbindung der Formel II, worin die Hydroxygruppe ungeschützt ist, bei der Thiolisierungsreaktion (Stufe a)) praktisch ausschliesslich die 3,4-trans-Verbindung der Formel IV liefert. Bisher war es immer üblich, diese Hydroxygruppe mit einer Schutzgruppe zu versehen, wobei aber in der nachfolgenden Thiolisierungsstufe als Nebenprodukt immer auch die 3,4-cis-Verbindung entsteht. Es ist also ein grosser Vorteil, dass die 1'-Hydroxygruppen in den Zwischenprodukten der Formeln II und IV nicht selektiv geschützt zu werden brauchen, etwa wie bisher immer üblich durch andere Acylgruppen oder durch Silylgruppen, sondern dass in einem Schritt die Verbindung der Formel IV sowohl am Azetidinonstickstoff mit den für den folgenden Ringschluss nötigen Acylrest des Oxalsäurehalbesters als auch an der Hydroxygruppe in der 1'-Stellung in schützender Weise mit dem gleichen Acylrest substituiert wird.

Es ist auch überraschend, dass das notwendigerweise als Zwischenprodukt entstehende Phosphoran nur am $\beta$-Lactam-N-benachbartem Carbonyl gebildet wird (Stufe c)) und dass die theoretisch mögliche Reaktion mit der Carbonylgruppe der 1'-Oxalylestergruppe nicht stattfindet.

Schliesslich beruht die erfindungsgemässe Verbesserung des Verfahrens auch auf den überraschenden Tatsachen, dass der Acylrest des Oxalsäurehalbesters der Formel V aus dem Zwischenprodukt der Formel VIII äusserst leicht, das heisst ohne Isomerisierung oder sonstige Zerstörung des Penemgerüstes, und, je nach Wunsch und den angewendeten Reaktionsbedingungen, entweder selektiv oder gleichzeitig mit den anderen Schutzgruppen $R_2^o$ oder $R_3^o$ abgespalten und durch Wasserstoff ersetzt werden kann.

Zahlreiche Penemverbindungen der Formel I sind bekannt, und die Substituenten $R_1$, $R_2$ und $R_3$ haben insbesondere die bekannten Bedeutungen.

Erfindungsgemäss herstellbare Verbindungen der Formel I sind beispielsweise in den folgenden Patentanmeldungen beschrieben:

Ciba-Geigy GB 2.013.676, EP 109 362, EP 110 826, EP 112 283, EP 125 208, EP 125 207, BE 9 00 477, EP 148 128; Beecham EP 46 363; Bristol Myers NL 7909 055, NL 7 909 056; USP 4,282,150; Farmitalia BE 881 862, GB 2 091 786, DE 3 312 393, BE 900 316; Hoechst EP 69 377, GB 2 122 619, GB 2 140 807; Merck EP 2 210, EP 72 014, EP 87 792, EP 115 308; Pfizer EP 130 025, EP 132 101; Sankyo BE 889 151, DE 3 231 596; Schering Corp. US 4 443 463, BE 881 012, US 4 423 055, EP 35 188, EP 58 317, US 4 431 654, US 4 435 412, US 4 435 413, US 4 503 064, EP 109 044, EP 118 875, EP 123 650, EP 121 502; Shionogi EP 115 969; Sumitomo EP 70 204, EP 126 587; Takeda EP 69 373.

Die in den Formeln I bis VIII auftretenden Substituenten $R_1$, $R_2$, $R_2^o$ , $R_3$, $R_3^o$ , W und Z haben insbesondere die folgenden Bedeutungen, wobei der im Zusammenhang mit Definitionen von Gruppen bzw. Verbindungen verwendete Ausdruck "Nieder" bedeutet, dass die entsprechenden Gruppen bzw. Verbindungen, sofern nicht ausdrücklich anders definiert, 1 bis 7, bevorzugt 1 bis 4 Kohlenstoffatome enthalten.

$R_1$ ist als Niederalkyl insbesondere Methyl, aber auch Ethyl, Propyl, Isopropyl oder Butyl. Bevorzugt sind solche Verbindungen, worin $R_1$ Wasserstoff oder insbesondere Methyl bedeutet.

$R_2$ ist insbesondere ein, z.B. in der Penemchemie bekannter organischer Rest mit bis zu 18, insbesondere bis zu 10, und bevorzugt bis zu 5 Kohlenstoffatomen, der bis zu 5, bevorzugt bis zu 4, Stickstoffatome, und/oder bis zu 5, bevorzugt bis zu 2 Sauerstoffatome, und/oder bis zu 3, bevorzugt 2 Schwefelatome oder insbesondere 1 Schwefelatom enthalten kann, und der sowohl über eines seiner Kohlenstoffatome als auch über ein Stickstoff-, Sauerstoff- oder Schwefelatom mit dem Penemring verbunden sein kann, und ist z.B. gegebenenfalls substituiertes Niederaliphatyl, Niederaliphatyloxy, Niederaliphatylthio, Cycloaliphatyl, Cycloaliphatyloxy, Cycloaliphatylthio, Aryl, Aryloxy, Arylthio, Arylniederaliphatyl, Arylniederaliphatyloxy, Arylniederaliphatylthio, Heterocyclyl, Heterocyclyloxy, Heterocyclylthio, Heterocyclylniederaliphatyl, Heterocyclylniederaliphatyloxy, Heterocyclylniederaliphatylthio, Heterocyclyloxyniederaliphatyl oder Heterocyclylthioniederaliphatyl, wobei Niederaliphatyl, Cycloaliphatyl und Heterocyclyl gesättigt oder ungesättigt sein können.

Niederaliphatyl ist z.B. Niederalkyl, ferner auch Niederalkenyl und Niederaliphatyloxy oder Niederaliphatylthio ist ein entsprechender über ein Sauerstoff- oder Schwefelatom gebundener Niederaliphatylrest.

Cycloaliphatyl ist beispielsweise Cycloalkyl oder ungesättigtes Cycloalkyl, wie Cycloalkenyl oder Cycloalkadienyl. Cycloaliphatyloxy oder Cycloaliphatylthio ist ein entsprechender über ein Sauerstoff- oder Schwefelatom gebundener Cycloaliphatylrest.

Aryl ist beispielsweise ein entsprechender monocyclischer oder auch polycyclischer, wie bicyclischer Rest, insbesondere Phenyl oder Naphthyl. Aryloxy oder Arylthio ist ein entsprechender über ein Sauerstoff- oder Schwefelatom gebundener Arylrest. Arylniederaliphatyl, Arylniederaliphatyloxy oder Arylniederaliphatylthio ist beispielsweise Phenylniederalkyl, Phenylniederalkoxy oder Phenylniederalkylthio.

Heterocyclyl, Heterocylyloxy oder Heterocyclylthio ist insbesondere ein entsprechender monocyclischer oder polycyclischer, insbesondere monocyclischer oder bicyclischer, ferner auch tricyclischer Rest, wie ein gegebenenfalls partiell gesättigter monocyclischer 5- oder 6-gliedriger Heteroarylrest mit mindestens einem Heteroatom aus der Gruppe Stickstoff, Sauerstoff und Schwefel, insbesondere ein solcher Rest mit 1-4 Ringstickstoffatomen und gegebenenfalls einem zusätzlichen Ringheteroatom aus der Gruppe Sauerstoff und Schwefel, z.B. ein entsprechender aza-, diaza-, triaza-, tetraza-, oxa-, oxaza-, oxadiaza-, oxatriaza-, thia-, thiaza-, thiadiaza- oder thiatriaza-cyclischer Rest aromatischen Charakters oder ein entsprechender Dihydro- oder Tetrahydrorest, ferner ein gegebenenfalls partiell gesättigtes Benzo-, Dibenzo-, Pyrido- oder Pyrimidoderivat eines solchen 5- oder 6-gliedrigen Restes. Entsprechende Heteroarylreste sind beispielsweise Pyrrolyl, wie 2- oder 3-Pyrrolyl, 2- oder 3-Pyrrolidinyl, Diazolyl, wie Imidazolyl, z.B. 1-, 2-, 4- oder 5-Imidazolyl, oder Pyrazolyl, z.B. 3- oder 4-Pyrazolyl, Triazolyl, z.B. 1,2,3-Triazol-4-yl, 1,2,4-Triazol-5-yl oder 1,2,4-Triazol-3-yl, Tetrazolyl, z.B. 1H- oder 2H-Tetrazol-5-yl oder -1-yl, Pyridyl, z.B. 2-, 3- oder 4-Pyridyl, Diazinyl, wie Pyrimidyl, z.B. 2-, 4- oder 5-Pyrimidyl oder Pyrazinyl, z.B. 2-Pyrazinyl, Triazinyl, z.B. 1,2,4-Triazin-6-yl oder 1,3,5-Triazin-2-yl, Furyl, z.B. 2- oder 3-Furyl, 2- oder 3-Tetrahydrofuryl, Thienyl, z.B. 2- oder 3-Thienyl, Oxazolyl, z.B. 2- oder 4-Oxazolyl, Oxadiazolyl, z.B. 1,2,4-Oxadiazol-3-yl, 1,2,5-Oxadiazol-3-yl, 1,2,4-Oxadiazol-5-yl oder 1,3,4-Oxadiazol-2-yl, Isoxazolyl, z.B. 3-Isoxazolyl, Thiazolyl, z.B. 2- oder 4-Thiazolyl, Thiadiazolyl, z.B. 1,2,4-Thiadiazol-3-yl, 1,2,5-Thiadiazol-3-yl, 1,2,4-Thiadiezol-5-yl oder 1,3,4-Thiadiazol-2-yl, oder Isothiazolyl, z.B. 3- oder 4-Isothiazolyl. Die genannten 5- und 6-gliedrigen Heteroarylreste können auch in partiell gesättigter Form vorliegen. Solche Reste sind beispielsweise Dihydroimidazolyl, z.B. 2,3-Dihydroimidazol-4-yl, Dihydropyridyl, z.B. 1,2-Dihydropyrid-3-yl, Dihydropyrimidyl, z.B. 1,2-Dihydropyrimid-4-yl, Dihydro- und Tetrahydropyrazinyl, z.B. 2,3-Dihydropyrazin-5-yl oder 3,4,5,6-Tetrahydropyrazin-2-yl, oder Dihydro- oder Tetrahydrotriazinyl, z.B. 2,3,4,5-Tetrahydro-1,2,4-triazin-6-yl. Polycyclische Derivate der genannten Heteroarylreste sind z.B. Indol-2- oder -3-yl, Benzimidazol-2-yl,

Benzopyrazol-3-yl, Chinolin-2-yl oder Chinolin-4-yl, Benzothiazol-2-yl, Benzoxazol-2-yl, Pyrido[2,3-b]-pyrid-3-yl oder Pyrido[2,3-b]pyrid-4-yl.

Heterocyclylniederaliphatyl, Heterocyclylniederaliphatyloxy oder Heterocyclylniederaliphatylthio, Heterocyclyloxyniederaliphatyl oder Heterocyclylthioniederaliphatyl sind z.B. Niederalkyl, Niederalkoxy oder Niederalkylthioreste, die durch einen oder zwei, insbesondere einen der obengenannten über eines seiner Kohlenstoffatome oder über ein Sauerstoff- oder Schwefelatom gebundenen Heterocyclylreste oder durch einen gegebenenfalls partiell gesättigten monocyclischen, 5- oder 6-gliedrigen, über eines seiner Ringstickstoffatome gebundenen Heteroarylrest, welcher als Ringheteroatome 1-4 Stickstoffatome enthält, substituiert sind. Bei den zuletzt genannten über ein Stickstoffatom gebundenen Resten handelt es sich beispielsweise um Imidazol-1-yl, Tetrahydro- oder 4,5-Dihydro-imidazol-1-yl, Pyrazol-1-yl, 1,2,4-Triazol-1-yl, 1,2,3-Triazol-1-yl, Tetrazol-1-yl, Tetrazol-2-yl, 1-Pyridinio, 1,2-Dihydro-1-pyridyl, 1,4-Dihydro-1-pyridyl, 1,2-Dihydro-pyrimid-1-yl oder 1,4-Dihydro-pyrimid-1-yl. Die Heterocyclylreste können auch über ein Sauerstoff- oder Schwefelatom an den niederaliphatischen Rest gebunden sein.

Die genannten organischen Reste $R_2$ sind unsubstituiert oder können z.B. durch gegebenenfalls veräthertes oder verestertes, inklusive geschütztes, Hydroxy, z.B. Hydroxy, Niederalkoxy, Phenylniederalkoxy, Niederalkanoyloxy oder Halogen; gegebenenfalls veräthertes Mercapto, z.B. Mercapto , Niederalkylthio oder Phenylthio; Niederalkyl; durch Hydroxy, Niederalkoxy, gegebenenfalls verestertes oder amidiertes Carboxy, wie Carboxy, Niederalkoxycarbonyl oder Carbamoyl, gegebenenfalls N-niederalkyliertes Amino, z.B. Amino, Niederalkylamino oder Diniederalkylamino, Niederalkylenamino, Niederalkanoylamino, gegebenenfalls durch Amino und/oder Carboxy substituiertes Niederalkoxycarbonylamino, Mercapto, Niederalkylthio oder Sulfo substituiertes Niederalkyl; Niederalkylen; gegebenenfalls niederalkyliertes Azaniederalkylen, Oxa-, Dioxa- oder Thianiederalkylen; gegebenenfalls substituiertes, inklusive geschütztes, Amino, z.B. Amino, Niederalkylamino, Diniederalkylamino, Niederalkylenamino, Guanidino, Acylamino, wie Niederalkanoylamino, oder gegebenenfalls durch Amino und/oder Carboxyl substituiertes Niederalkoxycarbonylamino; gegebenenfalls funktionell abgewandeltes, inklusive geschütztes Carboxyl oder Sulfo, z.B. Carboxyl, verestertes Carboxyl, wie Niederalkoxycarbonyl, amidiertes Carboxyl, wie gegebenenfalls substituiertes Carbamoyl, z.B. Carbamoyl oder N-mono-oder N,N-diniederalkyliertes Carbamoyl, Cyano, Sulfo oder Sulfamoyl; gegebenenfalls z.B. durch Niederalkyl, Nitro, Amino, Hydroxy, Niederalkoxy, Carboxy und/oder Halogen substituiertes Phenyl; Cycloalkyl; Nitro; Oxo und/oder Oxido substituiert, wie mono- oder auch poly-, wie insbesondere mono- oder di-, ferner auch trisubstituiert sein, wobei die Substituenten gleich oder verschieden sein können.

Die im Zusammenhang mit $R_2$ genannten Reste bzw. deren Substituenten haben beispielsweise die folgenden Bedeutungen:

Niederalkyl ist z.B. n-Propyl, Isopropyl, n-Butyl, Isobutyl, sek.-Butyl oder tert.-Butyl, ferner auch n-Pentyl, n-Hexyl oder n-Heptyl, in erster Linie jedoch Methyl oder Ethyl.

Niederalkenyl ist z.B. Vinyl, Allyl, 2-Methylallyl, n-Propenyl oder Isopropenyl.

Niederalkoxy ist z.B. Methoxy, ferner Ethoxy, n-Propyloxy, Isopropyloxy, n-Butyloxy, Isobutyloxy oder tert.-Butyloxy, sowie n-Pentyloxy, n-Hexyloxy oder n-Heptyloxy.

Niederalkylthio ist z.B. Methylthio oder Ethylthio, ferner auch n-Propylthio, Isopropylthio oder n-Butylthio.

Cycloalkyl enthält vorzugsweise 3-8, in erster Linie 5 oder 6 Ringglieder und ist zum Beispiel Cyclopentyl oder Cyclohexyl, ferner Cyclopropyl sowie Cycloheptyl, während Cycloalkenyl z.B. 5 oder 6 Ringglieder und Cycloalkadienyl z.B. 6 Ringglieder enthält und z.B. 1-, 2-oder 3-Cyclohexenyl, 1- oder 2-Cyclopentenyl oder 1,4-Cyclohexadienyl ist.

Phenylniederalkyl ist z.B. Benzyl, 1-Phenylethyl oder 2-Phenylethyl.

Phenylniederalkoxy ist z.B. Benzyloxy oder 1-Phenylethoxy.

Niederalkanoyloxy ist z.B. Formyloxy, Acetyloxy oder Propionyloxy.

Halogen ist z.B. Fluor, Chlor, Brom oder Jod.

Niederalkylamino ist z.B. Methylamino, Ethylamino, n-Propylamino, Isopropylamino oder n-Butylamino, während Diniederalkylamino z.B. Dimethylamino, Diethylamino, Di-n-propylamino oder Diisopropylamino bedeutet.

Niederalkylenamino weist insbesondere 4-6 Kohlenstoffatome auf und ist z.B. Pyrrolidino oder Piperidino.

Niederalkanoylamino ist z.B. Formylamino, Acetylamino oder Propionylamino.

Gegebenenfalls durch Amino und/oder Carboxy substituiertes Niederalkoxycarbonylamino ist z.B. Methoxycarbonylamino, Ethoxycarbonylamino oder Isopropoxycarbonylamino, ferner 2-Amino-2-carboxyethoxy-carbonylamino.

Niederalkylen als Substituent des Restes $R_2$ kann geradkettig oder verzweigtkettig sein und verbindet beispielsweise zwei vicinale Kohlenstoff- und/oder Heteroatome eines organischen Restes $R_2$ durch 3-5 Kohlenstoffatome. Solche Reste sind beispielsweise 1,3-Propylen, 1,4-Butylen, 1- oder 2-Methyl-1,3-propylen oder 2,2-Dimethyl-1,3-propylen.

Gegebenenfalls niederalkyliertes Azaniederalkylen, Oxaniederalkylen, Dioxaniederalkylen oder Thianiederalkylen ist insbesondere geradkettig, kann aber auch verzweigtkettig sein, und verbindet z.B. vicinale Kohlenstoff- und/oder Heteroatome im organischen Rest $R_2$ durch 3-5 Kohlenstoffatome. Solche Reste sind beispielsweise 1- oder 2-Aza-1,3-propylen, 1-Aza-1,4-butylen, 1-Methyl-1-aza, 1-Ethyl-1-aza-, 2-Methyl-2-aza- oder 2-Ethyl-2-aza-1,3-propylen oder 1-Methyl-1-aza- oder 2-Methyl-2-aza-1,4-butylen, ferner 1-Oxa-, 1,3-Dioxa-, 1-Thia- oder 1,3-Dithia-1,3-propylen, oder 1-Oxa-, 1,4-Dioxa-, 1-Thia- oder 1,4-Dithia-1,4-butylen.

N-mono-niederalkyliertes Carbamoyl ist z.B. N-Methyl-, N-Aethyl- oder N-Propylcarbamoyl, während N,N-Di-diniederalkyliertes Carbamoyl z.B. N,N-Dimethyl- oder N,N-Diethylcarbamoyl bedeutet.

Niederalkanoyl ist z.B. Formyl, Acetyl oder Propionyl; Niederalkenoyl ist z.B. Acryloyl; Cycloalkanoyl weist 5-7 Kohlenstoffatome auf und ist z.B. Cyclohexanoyl, während Phenylniederalkanoyl z.B. Phenylacetyl ist.

Niederalkoxysulfonyl ist z.B. Methoxysulfonyl oder Ethoxysulfonyl.

Gegebenenfalls durch Amino und Carboxy substituiertes Niederalkoxycarbonyl ist z.B. Methoxycarbonyl, Ethoxycarbonyl, n-Propoxycarbonyl oder 2-Amino-2-carboxyethoxycarbonyl.

Niederalkyl-thiocarbamoyl ist z.B. Methyl-thiocarbamoyl oder Ethylthiocarbamoyl.

Niederalkansulfonyl ist z.B. Methansulfonyl, Ethansulfonyl oder Propansulfonyl.

Geeignete niederaliphatische Reste $R_2$ sind z.B. Niederalkyl, z.B. Methyl, Ethyl, Isopropyl, Isobutyl oder sek.-Butyl, oder durch Hydroxy, Niederalkoxy, z.B. Methoxy oder Ethoxy, Niederalkanoyloxy, z.B. Acetyloxy, Carbamoyloxy, Halogen, z.B. Chlor oder Brom, Mercapto, Niederalkylthio, z.B. Methylthio, Amino, Niederalkylamino, z.B. Methyl- oder Ethylamino, Diniederalkylamino, z.B. Dimethylamino, Guanidino, Formamidino, Niederalkanoylamino, z.B. Acetylamino, durch Amino und Carboxy substituiertes Niederalkoxy-, wie Ethoxycarbonylamino, z.B. 2-Amino-2-carboxyethoxycarbonylamino, Carboxyl, Niederalkoxycarbonyl, z.B. Methoxy- oder Ethoxycarbonyl, Carbamoyl, Sulfo, Sulfamoyl und/oder Oxo substituiertes Niederalkyl, wie insbesondere Methyl, Ethyl, n-Propyl oder n-Butyl. Repräsentative Vertreter solcher niederaliphatischer Reste $R_2$ sind z.B. Methyl, Isopropyl, Isobutyl, sek.-Butyl, Hydroxymethyl, 1- oder 2-Hydroxyethyl, Mercaptomethyl, 2-Methylthioethyl, Aminomethyl, 1- oder 2-Aminoethyl, 1-, 2- oder 3-Aminopropyl, 1-, 2-, 3- oder 4-Aminobutyl, Guanidylmethyl, 1- oder 2-Guanidylethyl, 1-, 2- oder 3-Guanidylpropyl, Formamidinomethyl, Formamidino -1- oder -2-ethyl, oder Formamidino -1-, -2- oder -3-propyl, Carboxymethyl, Carbamoylmethyl, 2-Carboxyethyl, 2-Carbamoylethyl, Carbamoyloxymethyl, 1-, 2- oder 3-Carbamoyloxypropyl, Carboxyl oder Niederalkoxycarbonyl, wie Methoxycarbonyl.

Arylniederaliphatylreste $R_2$ sind beispielsweise gegebenenfalls substituiertes Phenylniederalkyl, wie insbesondere Phenyl-methyl oder -ethyl, worin die Substituenten, z.B. Niederalkyl, wie Methyl, Nitro, Hydroxy, Niederalkoxy, z.B. Methoxy oder Ethoxy, Carboxy und/oder Halogen, z.B. Chlor, sind.

Geeignete Cycloaliphatylreste $R_2$ sind z.B. Cycloalkenyl, z.B. 1- oder 2-Cyclohexenyl, oder Cyclohexadienyl, z.B. 1,4-Cyclohexadienyl.

Arylreste $R_2$ sind z.B. Phenyl oder durch Amino, Niederalkylamino, z.B. Methyl- oder Ethylamino, Diniederalkylamino, z.B. Dimethylamino, Niederalkyl, z.B. Methyl, Aminoniederalkyl, z.B. 2-Aminoethyl, Niederalkylaminoniederalkyl, z.B. 2-Methylaminoethyl, durch Amino und Carboxy substituiertes Niederalkoxycarbonylaminoniederalkyl, z.B. 2-(2-Amino-2-carboxyethoxycarbonylamino)ethyl, durch Amino und Carboxy substituiertes Niederalkoxycarbonylamino, z.B. 2-Amino-2-carboxyethoxycarbonylamino, Nitro, Hydroxy, Niederalkoxy, z.B. Methoxy oder Ethoxy, Halogen, z.B. Chlor oder Brom, oder Carboxy substituiertes, wie insbesondere mono-, aber auch disubstituiertes Phenyl.

Heterocyclylreste $R_2$ sind insbesondere gegebenenfalls z.B. durch Niederalkyl substituiertes Pyrrolyl oder Dihydropyrrolyl, z.B. 1-Methyl-2-pyrrolyl oder 4,5-Dihydro-3-pyrrolyl, oder gegebenenfalls durch Oxo substituiertes Pyrrolidyl, z.B. 2- oder 3-Pyrrolidyl oder 2-Oxo-3- oder -5-pyrrolidyl, gegebenenfalls z.B. durch Carboxy, Niederalkyl, Aminoniederalkyl oder Amino substituiertes Diazolyl, wie Imidazolyl, z.B. 1-, 2-, 4- oder 5-Imidazolyl, 4- oder 5-Methyl- oder 4,5-Dimethyl-1-imidazolyl oder 2-Amino-4-imidazolyl, oder Pyrazolyl, z.B. 3-Pyrazolyl, gegebenenfalls z.B. durch Niederalkyl, Amino, Carboxyniederalkyl und/oder Phenyl substituiertes Triazolyl, wie 1H-1,2,3-Triazol-4-yl, 1H-1,2,4-Triazol-3-yl oder 1H-1,2,4-Triazol-5-yl, z.B. die entsprechenden unsubstituierten Reste, 1-Methyl-1H-1,2,3-triazol-4-yl, 5-Methyl- oder 5-Amino-1H-1,2,4-triazol-3-yl, 3-Methyl-1-phenyl-1H-1,2,4-triazol-5-yl, oder 5-Carboxymethyl- oder 5-Phenyl-1H-1,2,4-triazol-3-yl, gegebenenfalls z.B. durch Niederalkyl, Carboxyniederalkyl, Sulfoniederalkyl, Diniederalkylaminoniederalkyl oder gegebenenfalls Substituenten, wie Halogen, enthaltendes Phenyl substituiertes Tetrazolyl, wie 1H-Tetrazol-1-yl, 1H- oder 2H-Tetrazol-5-yl, z.B. 1H-Tetrazol-5-yl, 1-Carboxymethyl-, 1-(2-Carboxyethyl)-, 1-(2-Sulfoethyl)-, 1-(2-Dimethylaminoethyl)- oder 1-Phenyl-1H-tetrazol-5-yl, gegebenenfalls z.B. durch Niederalkyl oder Amino substituiertes Thiazolyl, wie 2- oder 4-Thiazolyl, oder Isothiazolyl, wie 3-Isothiazolyl, z.B. 2-, 4- oder 5-Thiazolyl, 4-Methyl-5- oder 5-Methyl-4-thiazolyl, 2-Amino-4-thiazolyl, 2-Amino-4-methyl-5-thiazolyl, 4,5-Dimethyl-2-thiazolyl oder 3-Isothiazolyl, gegebenenfalls z.B. durch Niederalkyl, Hydroxy, Amino oder Niederalkylamino substituiertes Thiadiazolyl, wie 1,2,5-Thiadiazol-3-yl, 1,3,4-Thiadiazol-2-yl, 1,2,4-Thiadiazol-3-yl oder 1,2,4-Thiadiazol-5-yl, z.B. die entsprechenden unsubstituierten Gruppen, ferner 5-Methyl- oder 5-Amino-1,3,4-thiadiazol-2-yl, 3-Methyl-1,2,4-thiadiazol-5-yl, 5-Amino- oder 5-Methylamino-1,2,4-thiadiazol-3-yl oder 4-Hydroxy-1,2,5-thiadiazol-3-yl, gegebenenfalls z.B. durch Amino, Niederalkyl und/oder Phenyl substituiertes Oxazolyl oder Isoxazolyl, wie 2- oder 4-Oxazolyl oder 3-Isoxazolyl, z.B. die entsprechenden unsubstituierten Gruppen, ferner 4-Methyl-2-oxazolyl, 4,5-Diphenyl-2-oxazolyl, 2-Amino-4-oxazolyl oder 5-Methyl-3-isoxazolyl, gegegenenfalls z.B. durch Niederalkyl, Amino oder gegebenenfalls z.B.durch Nitro substituiertes Phenyl substituiertes Oxadiazolyl, wie 1,2,4-Oxadiazol-5-yl, 1,2,4-Oxadiazol-3-yl oder 1,3,4-Oxadiazol-2-yl, z.B. die entsprechenden unsubstituierten Gruppen, ferner 5-Methyl-1,3,4-oxadiazol-2-yl, 5-Phenyl-1,3,4-oxadiazol-2-yl oder 5-(4-Nitrophenyl)-1,3,4-oxadiazol-2-yl, gegebenenfalls z.B. durch Halogen, Niederalkyl oder Aminoniederalkyl substituiertes Furyl oder Thienyl, wie 2- oder 3-Furyl oder 2- oder 3-Thienyl, z.B. die entsprechenden unsubstituierten Gruppen, 5-Methyl- oder 5-Aminomethyl-2-furyl, oder 5-Aminomethyl-2-thienyl, durch Oxo substituierter Tetrahydrofuryl, z.B. 2-Oxotetrahydrofur-5-yl, gegebenenfalls z.B. durch Hydroxy, Halogen, Niederalkyl, Amino und/oder Oxido substituiertes Pyridyl, wie 2-, 3- oder 4-Pyridyl, z.B. die entsprechenden unsubstituierten Gruppen, 1-Oxido-2-pyridyl oder 4-Chlor-1-oxido-2-pyridyl, oder gegebenenfalls z.B. durch Niederalkyl, Amino, Diniederalkylamino, Oxo und/oder Carboxy substituiertes 1,2-Dihydropyrimidyl, wie 1,2-Dihydro-4-pyrimidyl, z.B. 2-Oxo-1,2-dihydro-4-pyrimidyl, 6-Methyl-, 5-Methyl-, 6-Amino-, 6-Dimethylamino-, 5-Carboxy- oder 6-Carboxy-2-oxo-1,2-dihydro-4-pyrimidyl.

Heterocyclylniederaliphatylreste $R_2$ sind z.B. gegebenenfalls substituierte Heterocyclylmethyl-, -ethyl- oder -propylreste, wie z.B. gegebenenfalls z.B. durch Niederalkyl oder Halogen substituiertes Pyrrol- oder Dihydropyrrol-1-ylmethyl, z.B. Pyrrol-, 3-Methyl-pyrrol-, 3,4-Dichlor-pyrrol-, 2,3- oder 2,5-Dihydropyrrol-1-ylmethyl, gegebenenfalls durch Oxo substituiertes Pyrrolidylmethyl, z.B. 2- oder 3-Pyrrolidylmethyl oder 2-Oxo-3- oder 2-Oxo-5-pyrrolidylmethyl, gegebenenfalls z.B. durch Niederalkyl substituiertes Diazolylmethyl, wie Imidazol-1-ylmethyl, Imidazol-4-ylmethyl oder Pyrazol-1-ylmethyl, gegebenenfalls z.B. durch Niederalkyl, Carboxyniederalkyl, Amino und/oder Phenyl substituiertes Triazolylmethyl, wie 1H-1,2,3-Triazol-1-ylmethyl, 1H-1,2,4-Triazol-1-ylmethyl oder 1H-1,2,4-Triazol-3-ylmethyl, z.B. die entsprechenden unsubstituierten Reste, 4- oder 5-Methyl-1,2,3-triazol-1-ylmethyl, 5-Methyl-, 5-Amino oder 5-Phenyl-1H-1,2,4-triazol-3-ylmethyl, gegebenenfalls z.B. durch Niederalkyl, Carboxyniederalkyl, Sulfoniederalkyl, Diniederalkylaminoniederalkyl, Amino oder gegebenenfalls durch Halogen substituiertes Phenyl substituiertes Tetrazolylmethyl, wie 1H-Tetrazol-1-ylmethyl, -eth-1- oder -2-yl oder -prop-1-, -2- oder -3-yl, 2H-Tetrazol-2-ylmethyl oder 1H-Tetrazol-5-ylmethyl, z.B. die entsprechenden unsubstituierten Reste, 5-Amino-, 5-Carboxymethyl-, 5-(2-Carboxyethyl)-, 5-Sulfomethyl-, 5-(2-Dimethylaminoethyl)- oder 5-Phenyl-1H-tetrazol-1-ylmethyl, 5-Amino-, 5-Carboxymethyl-, 5-Sulfomethyl-, 5-(2-Dimethylaminoethyl)- oder 5-Phenyl-2H-tetrazol-2-ylmethyl, oder 1-(2-Carboxyethyl)- oder 1-(2-Dimethylaminoethyl)-2H-tetrazol-5-ylmethyl, unsubstituiertes oder insbesondere durch Oxo und gegebenenfalls zusätzlich z.B. durch Halogen substituiertes Pyridyl- oder Dihydropyridylmethyl, wie Pyridiniomethyl, 2-, 3- oder 4-Pyridylmethyl oder 1,2- oder 1,4-Dihydropyrid-1-ylmethyl, z.B. 2-Oxo-1,2-dihydropyrid-1-ylmethyl oder 4-Oxo-1,4-dihydropyrid-1-ylmethyl, gegebenenfalls insbesondere durch Oxo und gegebenenfalls zusätzlich z.B. durch Niederalkyl, Amino, Diniederalkylamino oder Carboxy substituiertes Dihydropyrimid-1-ylmethyl, wie 2H-1,2-Dihydro- oder 4H-1,4-Dihydro-pyrimid-1-ylmethyl, z.B. 2-Oxo-1,2-dihydro-pyrimid-1-ylmethyl, 6-Methyl-, 5-Methyl-, 6-Amino-, 6-Dimethylamino-, 5-Carboxy- oder 6-Carboxy-2-oxo-1,2-dihydro-pyrimid-1-ylmethyl oder 4-Oxo-1,4-dihydro-pyrimid-1-ylmethyl, Furylmethyl, z.B. 2-Furylmethyl, gegebenenfalls durch Oxo substituiertes Tetrahydrofurylmethyl, z.B. 2-Oxotetrahydrofur-5-ylmethyl, Thienylmethyl, z.B. 2-Thienylmethyl, gegebenenfalls durch z.B. Amino substituiertes Oxazolylmethyl, Thiazolylmethyl oder Thiadiazolylmethvl, z.B. 2-Amino-oxazol-4-ylmethyl, 2-Amino-thiazol-4-ylmethyl oder 5-Amino-1,2,4-thiadiazol-3-ylmethyl, oder Indolylmethyl, z.B. Indol-3-ylmethyl.

Besonders bevorzugt sind solche Reste $R_2$ die in antibiotisch besonders aktiven Verbindungen der Formel I vorkommen.

Solche Reste $R_2$ sind beispielsweise Aminomethyl, 2-Aminoprop-1-yl, Carbamoyloxymethyl, 1-Methyltetrazol-5-ylthiomethyl, 2-(1-Tetrazolyl)propyl, 2-Oxo-5-tetrahydrofurylmethyl, 2-Oxo-5-pyrrolidylmethyl, 2-Aminoethylthio, 2-Formamidinoethylthio, 2-Carbamoyloxyethylthio, 1-Imidazolyl, 4,5-Dimethyl-1-imidazolyl, 4-Methyl-5-thiazolyl, 5-Methyl-4-thiazolyl und 2-Amino-4-methyl-5-thiazolyl, und ihre geschützten Derivate.

Die in den vorliegenden Verbindungen vorhandenen funktionellen Gruppen, wie Hydroxy-, Carboxy-, Amino- oder Sulfogruppen sind gegebenenfalls durch übliche Schutzgruppen $R_2^o$ und/oder $R_3^o$ geschützt. Uebliche Schutzgruppen sind insbesondere solche, die in der Penem-, Penicillin-, Cephalosporin- und Peptidchemie verwendet werden können. Solche Schutzgruppen schützen die betreffenden funktionellen Gruppen vor unerwünschten Eliminations- und Substitutionsreaktionen und dergleichen während der Synthese der Verbindung der Formel I aus ihren Vorstufen und können ohne Zerstörung des Penemringsystems abgespalten werden.

Solche Schutzgruppen sind leicht, d.h. ohne dass unerwünschte Nebenreaktionen stattfinden, beispielsweise solvolytisch, reduktiv oder auch unter physiologischen Bedingungen, abspaltbar.

Schutzgruppen dieser Art sowie ihre Einführung und Abspaltung sind beispielsweise beschrieben in
J.F.W. McOmie, "Protective Groups in Organic Chemistry", Plenum Press, London, New York, 1973,
T.W. Greene, "Protective Groups in Organic Synthesis", Wiley, New York, 1981,
"The Peptides", Vol. I, Schroeder und Luebke, Academic Press, London, New York, 1965 und
Houben-Weyl, "Methoden der Organischen Chemie", Band 15/1, Georg Thieme Verlag, Stuttgart, 1974.

In Verbindungen der Formel I kann eine Hydroxygruppe im Rest $R_2$ beispielsweise durch Acylreste geschützt sein. Geeignete Acylreste sind z.B. gegebenenfalls durch Halogen substituiertes Niederalkanoyl, z.B. Acetyl oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromethoxycarbonyl oder 2,2,2-Trichlorethoxycarbonyl, Niederalkenyloxycarbonyl, z.B. Allyloxycarbonyl, gegebenenfalls im Arylring substituiertes Arylniederalkenyloxycarbonyl, z.B. 3-Phenylallyloxycarbonyl (Cinnamyloxycarbonyl), worin die Aryl- bzw. Phenylgruppe z.B. durch Niederalkoxy, wie Methoxy, Halogen, wie Chlor oder Fluor, und/oder Nitro ein-, zwei- oder mehrfach substituiert sein kann, oder gegebenenfalls durch Nitro substituiertes Phenylniederalkoxycarbonyl, z.B. 4-Nitrobenzyloxycarbonyl. Weitere geeignete Hydroxyschutzgruppen sind z.B. trisubstituiertes Silyl, wie Triniederalkylsilyl, z.B. Trimethylsilyl oder tert.-Butyl-dimethylsilyl, 2-Halogenniederalkylgruppen, z.B. 2-Chlor-, 2-Brom-, 2-Jod- und 2,2,2-Trichlorethyl, und gegebenenfalls durch Halogen, z.B. Chlor, Niederalkoxy, z.B. Methoxy, und/oder Nitro substituiertes Phenylniederalkyl, wie entsprechendes Benzyl. Bevorzugt als Hydroxyschutzgruppe ist Triniederalkylsilyl, Niederalkenyloxycarbonyl und durch Halogen substituiertes Niederalkoxycarbonyl.

Eine Carboxylgruppe im Rest $R_2$, wie auch die Carboxylgruppe in 3-Stellung des Penemringes oder am Oxalsäurehalbester in 1'-Stellung, ist üblicherweise in veresterter Form geschützt, wobei die Estergruppe unter schonenden Bedingungen, z.B. unter schonend reduktiven, wie hydrogenolytischen, oder schonend solvolytischen, wie acidolytischen oder insbesondere basisch oder neutral hydrolytischen Bedingungen, leicht spaltbar ist. Eine geschützte Carboxylgruppe kann ferner eine leicht in eine andere funktionell abgewandelte Carboxylgruppe, wie in eine andere veresterte Carboxylruppe umwandelbare, veresterte Carboxylgruppe darstellen.

Solche veresterten Carboxylgruppen enthalten als veresternde Gruppen, z.B. $R_3^o$ , in erster Linie in 1-Stellung verzweigte oder in 1- oder 2-Stellung geeignet substituierte Niederalkylgruppen. Bevorzugte in veresterter Form vorliegende Carboxylgruppen, z.B. auch $-COOR_3^o$ , sind unter anderen Niederalkoxycarbonyl, z.B. Methoxycarbonyl, Ethoxycarbonyl, Isopropoxycarbonyl oder tert.-Butoxycarbonyl, und Aryl- oder Heteroarylmethoxycarbonyl mit 1 bis 3 Arylresten oder einem monocyclischen Heteroarylrest, wobei diese gegebenenfalls z.B. durch Niederalkyl, wie tert.-Niederalkyl, z.B. tert.-Butyl, Halogen, z.B. Chlor, und/oder Nitro mono- oder polysubstituiert sind. Beispiele für solche Gruppen sind gegebenenfalls z.B. wie oben erwähnt, substituiertes Benzyloxycarbonyl, z.B. 4-Nitrobenzyloxycarbonyl, gegebenenfalls, z.B. wie oben erwähnt, substituiertes Diphenylmethoxycarbonyl, z.B. Diphenylmethoxycarbonyl, oder Triphenylmethoxycarbonyl, oder gegebenenfalls, z.B. wie oben erwähnt, substituiertes Picolyloxycarbonyl, z.B. 4-Picolyloxycarbonyl, oder Furfuryloxycarbonyl, wie 2-Furfuryloxycarbonyl. Weitere geeignete Gruppen sind Niederalkanoylmethoxycarbonyl, wie Acetonyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, Halogenniederalkoxycarbonyl, wie 2-Halogenniederalkoxycarbonyl, z.B. 2,2,2-Trichlorethoxycarbonyl, 2-Chlorethoxycarbonyl, 2-Bromethoxycarbonyl oder 2-Jodethoxycarbonyl, oder ω-Halogenniederalkoxycarbonyl, worin Niederalkoxy 4-7 Kohlenstoffatome enthält, z.B. 4-Chlorbutoxycarbonyl, Phthalimidomethoxycarbonyl, Niederalkenyloxycarbonyl, z.B. Allyloxycarbonyl, gegebenenfalls im Arylring substituiertes Arylniederalkenyloxycarbonyl, z.B. 3-Phenylallyloxycarbonyl (Cinnamyloxycarbonyl), worin die Aryl- bzw. Phenylgruppe z.B. durch Niederalkoxy, wie Methoxy, Halogen, wie Chlor oder Fluor, und/oder Nitro ein-, zwei- oder mehrfach substituiert sein kann, oder in 2-Stellung durch Niederalkylsulfonyl, Cyano oder trisubstituiertes Silyl, wie Triniederalkylsilyl oder Triphenylsilyl, substituiertes Ethoxycarbonyl, z.B. 2-Methylsulfonylethoxycarbonyl, 2-Cyanoethoxycarbonyl, 2-Trimethylsilylethoxycarbonyl oder 2-(Di-n-butyl-methyl-silyl)-ethoxycarbonyl.

Eine Carboxylschutzgruppe ist auch eine unter physiologischen Bedingungen spaltbare veresterte Carboxylgruppe. Sie schützt die Verbindungen der Formel I vor Salzbildung im Magen-Darm-Trakt bei oraler Verabreichung, womit die vorzeitige Exkretion verhindert wird, und ist in erster Linie eine Acyloxymethoxycarbonylgruppe oder auch Acylaminomethoxycarbonylgruppe, worin Acyl z.B. den Rest einer organischen Carbonsäure, in erster Linie einer gegebenenfalls, z.B. durch Amino, substituierten Niederalkancarbonsäure oder Arylcarbonsäure, z.B. Benzoesäure, bedeutet oder worin Acyloxymethyl den Rest eines Lactons bildet. Solche Gruppen sind z.B. Niederalkanoyloxymethoxycarbonyl, Aminoniederalkanoyloxymethoxycarbonyl, insbesondere $\alpha$-Aminoniederalkanoyloxymethoxycarbonyl, Niederalkanoylaminomethoxycarbonyl, Benzaminomethoxycarbonyl, 4-Crotonolactonyl und 4-Butyrolacton-4-yl. Weitere unter physiologischen Bedingungen spaltbare veresterte Carboxylgruppen sind z.B. 5-Indanyloxycarbonyl, 3-Phthalidyloxycarbonyl, 1-Niederalkoxycarbonyloxyniederalkoxycarbonyl, 1-Niederalkoxyniederalkoxycarbonyl oder 2-Oxo-1,3-dioxolen-4-ylmethoxycarbonyl, welches in 5-Stellung des Dioxolenringes gegebenenfalls durch Niederalkyl oder Phenyl substituiert ist.

Weitere in veresterter Form vorliegende geschützte Carboxylgruppen sind entsprechende organische Silyloxycarbonyl-, ferner entsprechende organische Stannyloxycarbonylgruppen. In diesen enthält das Silicium- bzw. Zinnatom vorzugsweise Niederalkyl, insbesondere Methyl oder Ethyl, ferner Niederalkoxy, z.B. Methoxy, als Substituenten. Geeignete Silyl- bzw. Stannylgruppen sind in erster Linie Triniederalkylsilyl, insbesondere Trimethylsilyl oder Dimethyltert.-butylsilyl, oder entsprechend substituierte Stannylgruppen, z.B. Tri-n-butylstannyl.

Bevorzugte geschützte Carboxylgruppen sind die 4-Nitrobenzyloxycarbonyl-, Niederalkenyloxycarbonyl-, insbesondere Allyloxycarbonyl-, und die in 2-Stellung durch Niederalkylsulfonyl, Cyano oder Triniederalkylsilyl, z.B. Trimethylsilyl oder Di-n-butylmethylsilyl, substituierte Ethoxycarbonylgruppe.

Eine geschützte Aminogruppe kann beispielsweise in Form einer leicht spaltbaren Acylamino-, Acylimino-, verätherten Mercaptoamino-, Silyl- oder Stannylaminogruppe oder als Enamino-, Nitro-oder Azidogruppe vorliegen.

In einer entsprechenden Acylaminogruppe ist Acyl beispielsweise der Acylrest einer organischen Säure mit z.B. bis zu 18 Kohlenstoffatomen, insbesondere einer gegebenenfalls z.B. durch Halogen oder Phenyl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Fluor-, 2-Brom-, 2-Jod-, 2,2,2-Trifluor- oder 2,2,2-Trichloracetyl, gegebenenfalls substituiertes Benzoyl, z.B. Benzoyl, Halogenbenzoyl, wie 4-Chlorbenzoyl, Niederalkoxybenzoyl, wie 4-Methoxybenzoyl, oder Nitrobenzoyl, wie 4-Nitrobenzoyl. Insbesondere ist auch Niederalkenyloxycarbonyl, z.B. Allyloxycarbonyl, gegebenenfalls im Arylring substituiertes Arylniederalkenyloxycarbonyl, z.B. 3-Phenylallyloxycarbonyl (Cinnamyloxycarbonyl), worin die Aryl- bzw. Phenylgruppe z.B. durch Niederalkoxy, wie Methoxy, Halogen, wie Chlor oder Fluor, und/oder Nitro ein-, zwei- oder mehrfach substituiert sein kann, oder gegebenenfalls in 1- oder 2-Stellung substituiertes Niederalkoxycarbonyl, wie Niederalkoxycarbonyl, z.B. Methoxy- oder Ethoxycarbonyl, gegebenenfalls substituiertes Benzyloxycarbonyl, z.B. Benzyloxycarbonyl oder 4-Nitrobenzyloxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Chlorethoxycarbonyl, 2-Bromethoxycarbonyl oder 2-Jodethoxycarbonyl, oder 2-(trisubstituiertes Silyl)-ethoxycarbonyl, wie 2-Triniederalkylsilylethoxycarbonyl, z.B. 2-Trimethylsilylethoxycarbonyl oder 2-(Di-n-butylmethyl-silyl)-ethoxycarbonyl, oder 2-Triarylsilylethoxycarbonyl, wie 2-Triphenylsilylethoxycarbonyl, geeignet.

In einer Acyliminogruppe ist Acyl beispielsweise der Acylrest einer organischen Dicarbonsäure mit z.B. bis zu 12 Kohlenstoffatomen, insbesondere einer entsprechenden aromatischen Dicarbonsäure, wie Phthalsäure. Eine solche Gruppe ist in erster Linie Phthalimino.

Eine verätherte Mercaptoaminogruppe ist in erster Linie eine gegebenenfalls durch Niederalkyl, wie Methyl oder tert.-Butyl, Niederalkoxy, wie Methoxy, Halogen, wie Chlor oder Brom, und/oder Nitro substituierte Phenylthioaminogruppe oder eine Pyridylthioaminogruppe. Entsprechende Gruppen sind beispielsweise 2- oder 4-Nitrophenylthioamino oder 2-Pyridylthioamino.

Eine Silyl- oder Stannylaminogruppe ist in erster Linie eine organische Silyl- bzw. Stannylaminogruppe, worin das Silicium- bzw. Zinnatom vorzugsweise Niederalkyl, z.B. Methyl, Aethyl, n-Butyl oder tert.-Butyl, ferner Niederalkoxy, z.B. Methoxy, als Substituenten enthält. Entsprechende Silyl- oder Stannylgruppen sind in erster Linie Triniederalkysilyl, insbesondere Trimethylsilyl, ferner Dimethyl-tert.-butylsilyl, oder entsprechend substituiertes Stannyl, z.B. Tri-n-butylstannyl.

Weitere geschützte Aminogruppen sind z.B. Enaminogruppen, die an der Doppelbindung in 2-Stellung einen elektronenziehenden Substituenten, beispielsweise eine Carbonylgruppe, enthalten. Schutzgruppen

dieser Art sind beispielsweise 1-Acyl-niederalk-1-en-2-yl-reste, worin Acyl z.B. der entsprechende Rest einer Niederalkancarbonsäure, z.B. Essigsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters, z.B. -methylhalbesters oder -ethylhalbesters, und Niederalk-1-en insbesondere 1-Propen bedeutet. Entsprechende Schutzgruppen sind in erster Linie 1-Niederalkanoylprop-1-en-2-yl, z.B. 1-Acetyl-prop-1-en-2-yl, oder 1-Niederalkoxycarbonyl-prop-1-en-2-yl, z.B. 1-Ethoxycarbonyl-prop-1-en-2-yl.

Bevorzugte geschützte Aminogruppen sind z.B. Azido, Phthalimido, Nitro, Niederalkenyloxycarbonylamino, z.B. Allyloxycarbonylamino, Phenylniederalkenyloxycarbonylamino, z.B. 3-Phenylallyloxycarbonylamino (Cinnamyloxycarbonylamino) und gegebenenfalls durch Nitro substituiertes Benzyloxycarbonylamino. Ueberraschenderweise führt die Verwendung von Cinnamyloxycarbonyl als Schutzgruppe in Verbindungen worin $R_2$ eine geschützte Aminomethylgruppe bedeutet zu kristallinen Zwischenprodukten der Formeln I, IV, VII und VIII.

Eine geschützte Sulfogruppe im Rest $R_2$ ist in erster Linie eine veresterte, wie mit einem aliphatischen, cycloaliphatischen, cycloaliphatisch-aliphatischen, aromatischen oder araliphatischen Alkohol, z.B. einem Niederalkanol, oder mit einem Silyl- oder Stannylrest, wie Triniederalkysilyl, veresterte Sulfogruppe. In einer Sulfogruppe kann die Hydroxygruppe beispielsweise wie die Hydroxygruppe in einer veresterten Carboxylgruppe veräthert sein.

Die Bedeutung von $R_3^o$ als Carboxylschutzgruppe ist den oben definierten geschützten Carboxylgruppen zu entnehmen.

W in einer Verbindung der Formel II wird im Verfahren a) definiert.

Salze von erfindungsgemässen Verbindungen sind in erster Linie pharmazeutisch annehmbare, nichttoxische Salze von Verbindungen der Formel I. Solche Salze werden beispielsweise von den in Verbindungen der Formel I vorhandenen sauren Gruppen, z.B. Carboxyl- und Sulfogruppen, gebildet und sind in erster Linie Metall- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organischen Aminen, wie Niederalkylaminen, z.B. Triethylamin, Hydroxyniederalkylaminen, z.B. 2-Hydroxyethylamin, Bis-(2-hydroxyethyl)-amin oder Tris-(2-hydroxyethyl)-amin, basischen aliphatischen Estern von Carbonsäuren, z.B. 4-Aminobenzoesäure-2-diethylaminoethylester, Niederalkylenaminen, z.B. 1-Ethylpiperidin, Cycloalkylaminen, z.B. Dicyclohexylamin, oder Benzylaminen, z.B. N,N'-Dibenzylethylendiamin, Dibenzylamin oder N-Benzyl-$\beta$-phenethylamin. Verbindungen der Formel I mit einer basischen Gruppe, z.B. mit einer Aminogruppe, können Säureadditionssalze, z.B. mit anorganischen Säuren, wie Salzsäure, Schwefelsäure oder Phosphorsäure, oder mit geeigneten organischen Carbon- oder Sulfonsäure, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe können auch in Form von inneren Salze, d.h. in zwitterionischer Form, vorliegen.

Zur Isolierung oder Reinigung können auch pharmazeutisch ungeeignete Salze Verwendung finden. Zur therapeutischen Anwendung gelangen nur die pharmazeutisch annehmbaren, nicht-toxischen Salze, die deshalb bevorzugt sind.

Die einzelnen Stufen des erfindungsgemässen Verfahrens werden wie folgt durchgeführt.

Stufe a): Die Ausgangsverbindungen der Formel II sind bekannt (z.B. EP 82113, DE-OS 3 224 055, DE-OS 3 013 997 oder S. Hanessian et al., J. Am. Chem. Soc. 1985, 107, 1438-1439) oder können auf an sich bekannte Weise hergestellt werden. Ein gegen die Gruppe

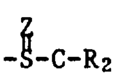

austauschbare Gruppe W ist eine nukleofuge Abgangsgruppe, z.B. ein Acyloxyrest, worin Acyl beispielsweise der Rest einer organischen Carbonsäure, wie Niederalkanoyl, z.B. Acetyl oder Propionyl, gegebenenfalls z.B. durch Nitro substituiertes Benzoyl, z.B. Benzoyl, 4-Nitro- oder 2,4-Dinitrobenzoyl, oder Phenylniederalkanoyl, z.B. Phenylacetyl, ein Sulfonylrest $R_o$-$SO_2$-, worin $R_o$ ein organischer Rest ist, und beispielsweise gegebenenfalls durch Hydroxy substituiertes Niederalkyl, z.B. Methyl, Ethyl, tert.-Butyl, 2-Hydroxyethyl, 1-Hydroxyprop-2-yl oder 1-Hydroxy-2-methylprop-2-yl, Benzyl oder gegebenenfalls z.B. durch Niederalkyl oder Halogen substituiertes Phenyl, z.B. Phenyl, 4-Bromphenyl oder 4-Methylphenyl darstellt, ein Halogenrest, z.B. Brom, Jod oder insbesondere Chlor, oder auch Azido. W ist bevorzugt Acetoxy, Benzoyloxy, Methan-, Benzol- oder Toluolsulfonyl, oder Chlor.

Die Ausgangsverbindungen der Formel II können als cis-, trans- oder Gemisch von cis-trans-Verbindungen eingesetzt werden, d.h. das $C_4$-Atom des Azetidinringes kann die R-, S- oder R,S-Konfiguration haben.

Den Rest der Formel III einführende Verbindungen sind die entsprechenden Thiocarbonsäuren H-S-C(=Z)-$R_2$ selbst oder Salze davon, z.B. Alkalimetallsalze, wie das Natrium- oder Kaliumsalz.

Die Substitution kann in einem organischen Lösungsmittel, wie in einem Niederalkanol, z.B. Methanol oder Ethanol, einem Niederalkanon, z.B. Aceton, einem Niederalkancarbonsäureamid, z.B. Dimethylformamid, einem cyclischen Ether, z.B. Tetrahydrofuran oder Dioxan, oder in einem ähnlichen inerten Lösungsmittel durchgeführt werden. Die Reaktion wird üblicherweise bei Raumtemperatur durchgeführt, kann aber auch bei erhöhter oder erniedrigter Temperatur, z.B. bei etwa 0° bis etwa 40°C, durchgeführt werden.

Die eintretende Gruppe wird von dem Rest $R_1$-CH(OH)- ausschliesslich in die trans-Stellung dirigiert.

Stufe b): In den Zwischenprodukten der Formel IV sind vorhandene funktionelle Gruppen im Rest $R_2$, z.B. Carboxyl-, Amino- oder Hydroxylgruppen, durch eine der genannten üblichen Schutzgruppen geschützt.

Ein den Acylrest einer Carbonsäure der Formel V einführendes Acylierungsmittel ist entweder die Carbonsäure der Formel V selbst oder eine reaktionsfähiges funktionelles Derivat oder ein Salz davon. Da sowohl die 1'-Hydroxygruppe als auch die Aminogruppe im Azetidinonring acyliert werden sollen, müssen mindestens zwei Aequivalente des Acylierungsmittels eingesetzt werden.

Falls eine freie Säure der Formel V zur Acylierung eingesetzt wird, wird die Reaktion üblicherweise in Gegenwart von geeigneten Kondensationsmitteln, wie Carbodiimiden, beispielsweise N,N'-Diethyl-, N,N'-Dipropyl-, N,N'-Dicyclohexyl- oder N-Ethyl-N'-3-dimethylaminopropylcarbodiimid, geeigneten Carbonylverbindungen, beispielsweise Carbonyldiimidazol, oder 1,2-Oxazoliumverbindungen, wie 2-Ethyl-5-phenyl-1,2-oxazolium-3'-sulfonat oder 2-tert.-Butyl-5-methyl-1,2-oxazoliumperchlorat, oder einer geeigneten Acylaminoverbindung, z.B. 2-Ethoxy-1-ethoxycarbonyl-1,2-dihydro-chinolin, durchgeführt.

Die Kondensationsreaktion wird vorzugsweise in einem wasserfreien Reaktionsmedium, vorzugsweise in einem inerten Lösungsmittel, z.B. Methylenchlorid, Dimethylformamid, Acetonitril oder Tetrahydrofuran, wenn erwünscht oder notwendig, unter Kühlen oder Erwärmen, z.B. in einem Temperaturbereich von etwa -40°C bis etwa +100°C, bevorzugt von etwa -20°C bis etwa +50°C, und, falls erforderlich, in einer Inertgas-, z.B. Stickstoffatmosphäre, durchgeführt.

Ein reaktionsfähiges, d.h. ein Carboxamid-bildendes, funktionelles Derivat einer Carbonsäure der Formel V ist in erster Linie ein Anhydrid, vorzugsweise ein gemischtes Anhydrid mit einer anorganischen Säure, z.B. einer Halogenwasserstoffsäure, und ist beispielsweise das entsprechendes Carbonsäurehalogenid, z.B. das Carbonsäurebromid oder insbesondere -chlorid, ferner das Carbonsäureazid. Weitere anorganische Säuren, die sich zur Bildung gemischter Anhydride eignen, sind Phosphor-haltige Säuren, z.B. Phosphorsäure, Diethylphosphorsäure oder phosphorige Säure, Schwefel-haltige Säuren, z.B. Schwefelsäure, oder Cyanwasserstoffsäure. Ein reaktionsfähiges, funktionelles Derivat einer Carbonsäure der Formel V wird ausserdem durch Kondensation mit einer organischen Carbonsäure gebildet, z.B. mit einer unsubstituierten oder durch Halogen, z.B. Fluor oder Chlor, oder Cyan substituierten Niederalkancarbonsäure, z.B. Pivalinsäure, Isovaleriansäure, Trifluoressigsäure oder Cyanessigsäure, mit einem Niederalkylhalbester der Kohlensäure, z.B. dem Ethyl- oder Isobutylhalbester der Kohlensäure, oder mit einer organischen, z.B. aliphatischen oder aromatischen Sulfonsäure, z.B. Methansulfonsäure oder p-Toluolsulfonsäure.

Ein reaktionsfähiges funktionelles Derivat einer Carbonsäure der Formel V ist ebenfalls ein aktivierter Ester, beispielsweise mit einem vinylogen Alkohol, d.h. einem Enol, z.B. einem vinylogen Niederalkenol, ein Iminomethylesterhalogenid, z.B. Dimethyliminomethylesterchlorid, hergestellt aus der Carbonsäure der Formel V und z.B. Dimethyl-(1-chlorethyliden)-iminiumchlorid der Formel $[(CH_3)_2N^{\oplus}=C(Cl)CH_3]Cl^{\ominus}$, das man seinerseits z.B. aus N,N-Dimethylacetamid und Phosgen oder Oxalylchlorid erhalten kann, ein Arylester, z.B. ein durch Halogen, z.B. Chlor, und/oder Nitro substituierter Phenylester, z.B. ein Pentachlor-, 4-Nitrophenyl- oder 2,4-Dinitrophenylester, ein N-heteroaromatischer Ester, z.B. N-Benztriazolester, oder ein N-Diacyliminoester, z.B. ein N-Succinimino- oder N-Phthaliminoester.

Bevorzugtes Acylierungsmittel sind die Säurehalogenide, z.B. die Chloride, insbesondere das Oxalsäureallylesterchlorid.

Die Acylierung mit einem reaktionsfähigen funktionellen Derivat der Carbonsäure der Formel V, insbesondere einem Säurehalogenid, wird bevorzugt in Anwesenheit eines geeigneten säurebindenden Mittels, beispielsweise einer geeigneten organischen Base, durchgeführt. Eine geeignete organische Base ist beispielsweise ein Amin, z.B. ein tertiäres Amin, wie Triniederalkylamin, z.B. Trimethylamin, Triäthylamin oder Aethyl-diisopropylamin, oder ein N,N-Diniederalkylanilin, z.B. N,N-Dimethylanilin, oder ein cyclisches tertiäres Amin, z.B. ein N-niederalkyliertes Morpholin, z.B. N-Methylmorpholin, oder ist beispielsweise eine Base vom Pyridin-Typ, z.B. Pyridin. Ein geeignetes säurebindendes Mittel ist ferner eine anorganische Base, beispielsweise ein Alkalimetall- oder Erdalkalimetallhydroxid, -carbonat oder -hydrogencarbonat, z.B.

Natrium-, Kalium- oder Calciumhydroxid, -carbonat oder -hydrogencarbonat.

Die Acylierung mit einem reaktionsfähigen, funktionellen Derivat der Carbonsäure der Formel V wird vorzugsweise in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, cyclischen Aether, z.B. Tetrahydrofuran, einem Ester, z.B. Essigsäureethylester, oder einem Nitril, z.B. Acetonitril, oder in Mischungen davon, wenn notwendig oder erwünscht, bei erniedrigter oder erhöhter Temperatur, z.B. in einem Temperaturbereich von etwa -40° bis etwa +100°C, bevorzugt von etwa -10° bis etwa +50°C, und, falls erforderlich, in einer Inertgas-, z.B. Stickstoffatmosphäre.

Ein bei der Acylierungsreaktion eingesetztes, reaktionsfähiges funktionelles Derivat einer Säure der Formel V kann in situ gebildet werden.

Stufe c): Eine organische Verbindung des dreiwertigen Phosphors leitet sich z.B. von phosphoriger Säure ab und ist insbesondere ein Ester derselben mit einem Niederalkanol, z.B. Methanol oder Ethanol, und/oder einer gegebenenfalls substituierten aromatischen Hydroxyverbindung, z.B. Phenol oder Brenzcatechin, oder ein Amidester derselben der Formel $P(OR_a)-N(R_b)_2$, worin $R_a$ und $R_b$ unabhängig voneinander Niederalkyl, z.B. Methyl, oder Aryl, z.B. Phenyl, bedeuten. Bevorzugte Verbindungen des dreiwertigen Phosphors sind Triniederalkylphosphite, z.B. Trimethylphosphit, Triethylphosphit oder Triisopropylphosphit.

Die Reaktion wird vorzugsweise in einem inerten Lösungsmittel, wie einem aromatischen Kohlenwasserstoff, z.B. Benzol oder Toluol, einem Ether, z.B. Dioxan oder Tetrahydrofuran, oder einem halogenierten Kohlenwasserstoff, z.B. Methylenchlorid oder Chloroform, bei einer Temperatur von etwa 20° bis 80°C, bevorzugt bei etwa 40° bis etwa 60°C, durchgeführt, wobei man 1 Moläquivalent einer Verbindung der Formel VI mit 2 Moläquivalenten der Phosphorverbindung umsetzt. Vorzugsweise legt man die Verbindung der Formel VII in einem inerten Lösungsmittel vor und tropft die Phosphorverbindung, vorzugsweise in dem gleichen inerten Lösungsmittel gelöst, über einen längeren Zeitraum, z.B. während eines Zeitraums von 2 bis 4 Stunden, hinzu und erhitzt auf etwa 100 bis 110° bis zur Beendigung der Reaktion.

In einer bevorzugten Ausführungsform des Verfahrens stellt man das Ausgangsmaterial der Formel VII wie oben angegeben her und setzt es ohne Isolierung aus dem Reaktionsgemisch mit der organischen Verbindung des dreiwertigen Phosphors um.

Stufe d): In einer erhältlichen Verbindung der Formel VIII, können die Schutzgruppen $R_3^o$, $R_3^o$ O-CO-CO-, und gegebenenfalls im Rest $R_2$ vorhandene Schutzgruppen $R_2^o$, z.B. aus geschützten Carboxyl-, Hydroxy-, Amino-, und/oder Sulfogruppen, in an sich bekannter Weise, z.B. mittels Solvolyse, insbesondere Hydrolyse, Alkoholyse oder Acidolyse, oder mittels Reduktion, insbesondere Hydrogenolyse oder chemische Reduktion, gegebenenfalls stufenweise und in beliebiger Reihenfolge oder gleichzeitig abgespalten und durch Wasserstoff ersetzt werden.

So kann die Oxalylestergruppe -CO-COO-$R_3$°, gegebenenfalls stufenweise und/oder selektiv, d.h. unter Erhaltung der Schutzgruppe $R_3^o$ an der 4-Carboxylgruppe oder anderer Schutzgruppen $R_2^o$, abgespalten und durch Wasserstoff ersetzt werden.

Die Abspaltung erfolgt beispielsweise unter alkoholischen Bedingungen, z.B. in Gegenwart von Alkalimetall-, wie Lithium-, Natrium- oder Kaliumhydroxiden, oder entsprechenden Carbonaten oder bevorzugt Hydrogencarbonaten, insbeondere von Natriumhydrogencarbonat, die bevorzugt in katalytischen oder bis zu äquivalenten Mengen eingesetzt werden. Als Lösungsmittel dienen Mischungen von Wasser mit wasserlöslichen organischen Lösungsmitteln, wie niederen Alkoholen, z.B. Methanol, Ethanol oder Isopropanol, niedere Ketone, z.B. Aceton, niedere cyclische Ether, z.B. Dioxan oder Tetrahydrofuran, niedere Nitrile, z.B. Acetonitril, niedere Amide, z.B. Dimethylformamid oder niedere Sulfoxide, z.B. Dimethylsulfoxid und dergleichen. Zur gegebenenfalls selektiven Abspaltung der 1'-Oxalylschutzgruppe wird bevorzugt in wässrigem Methanol, etwa 80 %ig, in Gegenwart von Natriumhydrogencarbonat verseift.

Die Temperaturen liegen zwischen dem Erstarrungspunkt und der Siedetempertur des verwendeten Lösungsmittels, etwa zwischen -20 bis +100°. Im allgemeinen wird bei tiefen Temperaturen, d.h. bei 0 bis 20°, bevorzugt bei etwa 0° gearbeitet.

Es kann auch zunächst eine Gruppe $R_3^o$ nach den unten angegebenen Methoden abgespalten werden, worauf der erhaltene 1'-Oxalsäurehalbester unter den oben genannten alkalischen Bedingungen vollständig verseift wird.

Eine geschützte Carboxylgruppe, z.B. eine Gruppe -COO-$R_3^o$, kann in an sich bekannter Weise freigesetzt werden. Beispielsweise kann man tert.-Niederalkoxycarbonyl oder in 2-Stellung durch eine trisubstituierte Silylgruppe oder in 1-Stellung durch Niederalkoxy substituiertes Niederalkoxycarbonyl oder gegebenenfalls substituiertes Diphenylmethoxycarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann

z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, oder Metallsalz, wie einem Chrom-II-Salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer geeigneten Carbonsäure, z.B. einer gegebenenfalls, z.B. durch Hydroxy, substituierten Niederalkancarbonsäure, z.B. Essigsäure, Ameisensäure oder Glykolsäure, oder eines Alkohols oder Thiols, wobei man vorzugsweise Wasser zugibt, in freies Carboxyl überführen. Die Abspaltung einer Allylschutzgruppe kann z.B. durch Umsetzung mit einer Verbindung des Palladiums, z.B. Tetrakis-(triphenylphosphin)-palladium, in Gegenwart eines Allylgruppenakzeptors, wie Acetylaceton oder insbesondere Dimedon, und gegebenenfalls von Triphenylphosphin und unter Zusatz einer Carbonsäure, z.B. 2-Ethylhexansäure, oder eines Salzes davon erfolgen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Aroylmethoxycarbonyl in freies Carboxyl umwandeln, während Aroylmethoxycarbonyl ebenfalls durch Behandeln mit einem nucleophilen, vorzugsweise salzbildenden Reagenz, wie Natriumthiophenolat oder Natriumjodid, gespalten werden kann. Substituiertes 2-Silylethoxycarbonyl kann auch durch Behandeln mit einem das Fluoridanion liefernden Salz der Fluorwasserstoffsäure, wie einem Alkalimetallfluorid, z.B. Natriumfluorid, in Anwesenheit eines makrocyclischen Polyethers ("Kronenäther") oder mit einem Fluorid einer organischen quaternären Base, wie Tetraniederalkylammoniumfluorid, z.B. Tetrabutylammoniumfluorid, in freies Carboxyl überführt werden. Mit einer organischen Silyl- oder Stannylgruppe, wie Triniederalkylsilyl oder -stannyl, verestertes Carboxyl kann in üblicher Weise solvolytisch, z.B. durch Behandeln mit Wasser oder einem Alkohol, freigesetzt werden. Eine in 2-Stellung durch Niederalkylsulfonyl oder Cyano substituierte Niederalkoxycarbonylgruppe kann z.B. durch Behandeln mit einem basischen Mittel, wie einem Alkalimetall- oder Erdalkalimetallhydroxid oder -carbonat, z.B. Natrium- oder Kaliumhydroxid oder Natrium- oder Kaliumcarbonat, in freies Carboxyl übergeführt werden.

In erhältlichen Verbindungen der Formel VIII, worin der Rest $R_2$ geschütztes Hydroxyl als Substituenten enthält, kann die geschützte Hydroxygruppe in an sich bekannter Weise in die freie Hydroxygruppe überführt werden. Beispielsweise wird eine durch eine geeignete Acylgruppe oder eine organische Silyl- oder Stannylgruppe geschützte Hydroxygruppe wie eine entsprechend geschützte Aminogruppe freigesetzt (s.u.); eine Triniederalkylsilylgruppe z.B. auch mit Tetrabutylammoniumfluorid und Essigsäure (unter diesen Bedingungen werden durch trisubstituiertes Silylethoxy geschützte Carboxygruppen nicht gespalten). Eine 2-Halogenniederalkylgruppe und eine gegebenenfalls substituierte Benzylgruppe werden reduktiv abgespalten.

In einer erhältlichen Verbindung der Formel VIII mit einer geschützten Aminogruppe kann diese in an sich bekannter Weise, z.B. je nach Art der Schutzgruppe, vorzugsweise mittels Solvolyse oder Reduktion, in die freie Aminogruppe übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe), Aroylmethoxycarbonylamino oder 4-Nitrobenzyloxycarbonylamino durch Behandeln mit einem geeigneten chemischen Reduktionsmittel, wie Zink in Gegenwart einer geeigneten Carbonsäure, wie wässriger Essigsäure, oder katalytisch mit Wasserstoff in Gegenwart eines Palladiumkatalysators gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z.B. Natriumdithionit gespalten werden. Gegebenenfalls substituiertes Benzyloxycarbonylamino kann z.B. mittels Hydrogenolyse, d.h. durch Behandeln mit Wasserstoff in Gegenwart eines geeigneten Hydrierkatalysators, wie eines Palladiumkatalysators, und Niederalkenyl- oder Arylniederalkenyloxycarbonylamino, wie Allyloxycarbonylamino oder Phenylallyloxycarbonylamino, durch Umsetzen mit einer Verbindung des Palladiums, z.B. Tetrakis(triphenylphosphin)palladium, in Gegenwart eines Allylgruppenakzeptors, wie Acetylaceton oder insbesondere Dimedon, und gegebenenfalls von Triphenylphosphin und Behandeln mit einer Carbonsäure, z.B. 2-Ethylhexansäure, oder eines Salzes davon gespalten werden. Eine mit einer organischen Silyl- oder Stannylgruppe geschützte Aminogruppe kann z.B. mittels Hydrolyse oder Alkoholyse, eine durch 2-Halogenniederalkanoyl, z.B. 2-Chloracetyl, geschützte Aminogruppe durch Behandeln mit Thioharnstoff in Gegenwart einer Base oder mit einem Thiolatsalz, wie einem Alkalimetallthiolat, des Thioharnstoffs und anschliessender Solvolyse, wie Alkoholyse oder Hydrolyse des entstandenen Kondensationsproduktes freigesetzt werden. Eine durch 2-substituiertes Silylethoxycarbonyl geschützte Aminogruppe kann durch Behandeln mit einem Fluoridanionen liefernden Salz der Fluorwasserstoffsäure, wie einem Alkalimetallfluorid, z.B. Natriumfluorid, in Anwesenheit eines makrocyclischen

Polyethers ("Kronenäther") oder mit einem Fluorid einer organischen quaternären Base, wie Tetraniederalkylammoniumfluorid, z.B. Tetraethylammoniumfluorid, in die freie Aminogruppe übergeführt werden. Eine in Form einer Azido- oder Nitrogruppe geschützte Aminogruppe wird z.B. durch Reduktion in freies Amino überführt, beispielsweise durch katalytische Hydrierung mit Wasserstoff in Gegenwart eines Hydrierkatalysators wie Platinoxid, Palladium oder Raney-Nickel, oder durch Behandeln mit Zink in Gegenwart einer Säure, wie Essigsäure. Eine in Form einer Phthalimidogruppe geschützte Aminogruppe kann durch Umsetzen mit Hydrazin in die freie Aminogruppe überführt werden. Weiterhin kann eine Arylthioaminogruppe durch Behandeln mit einem nucleophilen Reagenz, wie schwefliger Säure, in Amino umgewandelt werden.

Eine geschützte, insbesondere veresterte, Sulfogruppe wird analog einer geschützten Carboxylgruppe freigesetzt.

Salze von Verbindungen der Formel I mit salzbildenden Gruppen können in an sich bekannter Weise hergestellt werden. So kann man Salze von Verbindungen der Formel I mit einer freien Carboxyl- oder Sulfogruppe z.B. durch Behandeln mit Metallverbindungen, wie Alkalimetallsalzen von geeigneten organischen Carbonsäure, z.B. dem Natriumsalz der $\alpha$-Ethylcapronsäure, oder mit anorganischen Alkali- oder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze z.B. durch Behandeln mit geeigneten Säuren und Säureadditionssalze z.B. durch Behandeln mit einem geeigneten basischen Mittel.

Bei allen nachträglichen Umwandlungen erhaltener Verbindungen der Formel I werden solche Reaktionen bevorzugt, die unter schwach alkalischen oder insbesondere neutralen Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird, sowie die einzelnen Verfahrensstufen a) bis d). Ferner können Ausgangsstoffe in Form von Derivaten verwendet oder in situ, gegebenenfalls unter den Reaktionsbedingungen, hergestellt werden.

Die Erfindung betrifft auch die neuen Verbindungen der Formel I und IV, worin $R_2$ Cinnamyloxycarbonylaminomethyl ist.

Neue Verbindungen der Formel I sind insbesondere solche, worin $R_2$ Cinnamyloxycarbonylaminomethyl ist und $R_1$ und $R_3$ die andgegebenen Bedeutungen haben, wobei $R_1$ insbesondere Methyl und $R_3$ insbesondere Allyl ist.

Neue Verbindungen der Formel IV sind insbesondere solche, worin $R_2$ Cinnamyloxycarbonylaminomethyl ist und $R_1$ und Z die angegebenen, insbesondere bevorzugten Bedeutungen haben.

In den neuen Verbindungen der Formeln VII und VIII haben $R_2$ und $R_3^o$ die angegebenen, insbesondere bevorzugten Bedeutungen.

Bevorzugt sind insbesondere Verbindungen der Formeln I, IV, VII und VIII worin $R_2$ Cinnamyloxycarbonylaminomethyl ist in kristalliner Form.

Die folgenden Beispiele dienen zur Illustration der Erfindung. Temperaturen werden in Celsiusgraden angegeben.

In den Beispielen werden folgende Abkürzungen verwendet:

DC: Dünnschichtchromatogramm
IR: Infrarotspektrum
UV: Ultraviolettspektrum
THF: Tetrahydrofuran
F: Schmelzpunkt

Beispiel 1: (3S,4R,1'R)-3-(1'-Hydroxyethyl)-4-(N-allyloxycarbonylglycylthio)-2-azetidinon.

a) Eine Lösung von 14,2 g (40 mmol) N-Allyloxycarbonylthioglycindicyclohexylammoniumsalz in 60 ml Wasser und 40 ml 1N wässriger NaOH wird dreimal mit je 20 ml Methylenchlorid extrahiert und mit ca. 1 ml 0,1 N wässriger HCl der pH auf 7-8 gestellt. Die erhaltene wässrige Thiolsäurelösung wird bei 30° innert 5 Minuten zu einer Lösung von 4,70 g (20 mmol) (3R,4R,1'R)-3-(1'-Hydroxyethyl)-4-benzoyloxy-2-azetidinon in 100 ml Acetonitril gegeben. Bei einer Innentemperatur von 25° wird 2 ml 0,1N wässrige NaOH zugegeben und 30-35 Minuten bei 25° weitergerührt. Zur Aufarbeitung werden in einem Scheidetrichter 250 ml Essigsäureethylester und 30 g NaCl vorgelegt und dazu das Reaktionsgemisch gegeben. Nach gutem Schütteln und Abtrennen der wässrigen Phase wird die organische Phase noch einmal mit 50 ml 5%iger wässriger $NaHCO_3$-Lösung und zweimal mit 50 ml Sole gewaschen, über Natriumsulfat getrocknet. Das Lösungsmittel wird am Rotationsverdampfer entfernt. Man erhält die Titelverbindung als amorphes Pulver. Das Rohprodukt kann an Kieselgel chromatographisch gereinigt werden (Toluol/Essigester 2:3).

Rf-Wert 0.23 (Merck Fertigplatten, Toluol/Essigester = 1:4, Ninhydrin als Entwicklungsreagens).

Die gleiche Verbindung kann auch wie folgt erhalten werden:

b) Eine Lösung von 85,2 g (239 mmol) N-Allyloxycarbonylthioglycindicyclohexylammoniumsalz in 360 ml Wasser und 240 ml 1N wässriger NaOH wird dreimal mit je 40 ml $CH_2Cl_2$ extrahiert und mit 0,5 ml 1N wässriger HCl der pH-Wert der Wasserphase auf 7-8 gestellt. Die erhaltene wässrige Thiolsäurelösung wird bei 28° innert 5 Minuten zu einer Lösung von 30,1 g (128 mmol) (3S,4R,1'R)-3-(1'-Hydroxyethyl)-4-tert.-butylsulfonyl-2-azetidinon in 300 ml Acetonitril und 20 ml $H_2O$ zufliessen gelassen. Nach Zugabe von 12 ml 0,1 N wässriger NaOH wird 30 Minuten bei 25° weitergerührt. Zur Aufarbeitung werden in einem Scheidetrichter 700 ml Essigsäureethylester und 90 g NaCl vorgelegt und das Reaktionsgemisch dazugegeben. Nach gutem Schütteln und Abtrennen der organischen Phase wird diese noch einmal mit 100 ml 5%iger wässriger $NaHCO_3$-Lösung und zweimal mit 100 ml konz. wässriger Natriumchloridlösung gewaschen, über Natriumsulfat getrocknet und am Rotationsverdampfer das Lösungsmittel vollständig entfernt. Der teilweise kristalline Rückstand wird mit 100 ml $CH_2Cl_2$ versetzt, das kristalline, nicht umgesetzte Edukt abfiltriert und getrocknet. Das Filtrat wird eingedampft und an 300 g Kieselgel mit Toluol/Essigsäureethylester = 4:6 chromatographisch gereinigt. Man erhält die Titelverbindung vom Rf-Wert 0.23 (Merck Fertigplatten, Toluol/Essigsäureethylester 1:4, Ninhydrin als Entwicklungsreagenz).

c) Eine Lösung von 426 mg (1,2 mmol) N-Allyloxycarbonylthioglycindicyclohexylammoniumsalz in 1,2 ml 1N wässriger Natronlauge wird dreimal mit 1,5 ml $CH_2Cl_2$ extrahiert und mit 0,1 N wässriger Salzsäure der pH-Wert der Wasserphase auf 8-9 gestellt. Die erhaltene wässrige Thiolsäurelösung wird bei 25°C zu einer Lösung von 173,2 mg (1 mmol) (3S,4R,1'R)-3-(1'-Hydroxyethyl)-4-acetoxy-2-azetidinon in 1,7 ml Acetonitril zufliessen gelassen. Nach Zugabe von 0,1 ml 0,1 N wässriger Natronlauge wird 35 Minuten bei 21-23°C weitergerührt. Die Aufarbeitung wird wie im Beispiel 1a) durchgeführt. Nach chromatographischer Reinigung des Rohprodudkts (Toluol/Essigester 2:3) erhält man die Titelverbindung.

Beispiel 2: (5R,6S,1'R)-(1'-Hydroxyethyl)-2-allyloxycarbonylaminomethylpenem-3-carbonsäureallylester

Eine Lösung von 2,88 g (10,0 mmol) (3S,4R,1'R)-3-(1'-Hydroxyethyl)-4-(N-allyloxycarbonylglycylthio)-2-azetidinon (Rohprodukt vom Verfahren 1a) in 80 ml Methylenchlorid (frisch über Alox filtriert) wird unter Rühren und Feuchtigkeitsausschluss bei -10 bis -15°C nacheinander mit 3,86 ml (31,5 mmol) frisch destilliertem Oxalsäureallylesterchloridund 7,69 ml (44,8 mmol) N-Ethyl-diisopropylamin versetzt und 30 Minuten bei -10° weitergerührt. Eine IR-Probe zeigte die charakteristische Oxalimid-Carbonyl-Absorption bei 1820 $cm^{-1}$.

Zum Aufarbeiten wird das Reaktionsgemisch mit 50 ml $CH_2Cl_2$ verdünnt und dreimal mit eiskaltem Wasser und einmal mit Eiswasser und 5 ml gesättigter, wässriger $NaHCO_3$-Lösung gewaschen. Die wässrigen Anteile werden einmal mit $CH_2Cl_2$ nachextrahiert. Die vereinigten organischen Phasen werden mit $Na_2SO_4$ getrocknet, im Vakuum eingedampft und das Rohprodukt am Hochvakuum getrocknet. Der erhaltene dickflüssige Rückstand enthaltend den (3S,4R,1'R)-2-[4-(N-Allyloxycarbonylglycylthio)-3-(1'-allyloxyoxalyloxyethyl)-2-oxo-1-azetidinyl]-2-oxoessigsäureallylester (IR($CH_2Cl_2$) 3440 (NH); 1820

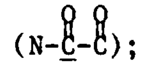

1720-1760

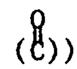

wird in 30 ml Dioxan gelöst und unter $N_2$-Atmosphäre bei Raumtemperatur mit 7 ml (40,2 mmmol) Triethylphosphit versetzt. Nach 15 Stunden bei Raumtemperatur kann im IR kein Oxalimid mehr festgestellt werden (Bande bei 1820 $cm^{-1}$). Das Reaktionsgemisch wird am Vakuum eingedampft, der Rückstand dreimal mit 2 ml Toluol und 2 ml Decan versetzt und die Schleppmittel zur Entfernung überschüssigen Phosphits/Phosphats jeweils am Hochvakuum wieder abgezogen. Das erhaltene rohe Phosphoran wird zur Cyclisierung in 250 ml Dioxan gelöst und 5 Stunden bei 105-110° gerührt. Das Reaktionsgemisch, enthaltend den (5R,6S,1'R)-2-Allyoxycarbonylaminomethyl-6-[1'-(allyloxyoxalyloxy)ethyl]-2-penem-3-carbonsäureallylester (für analytische Zwecke wird eine Probe des rohen Cyclisierungsproduktes an Kieselgel mit Toluol/Essigsäureethylester 4:1 gereinigt: UV (EtOH) 315 nm; IR($CH_2Cl_2$) 3440 (NH); 1790, 1770, 1745, 1720

( 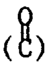 ))

wird im Vakuum auf ca 15 ml eingedampft, mit 100 ml Methanol/$H_2O$ 8:2 und mit 25 ml gesättigter wässriger $NaHCO_3$-Lösung bei 0° versetzt. Nach 20 Minuten wird das Reaktionsgemisch auf 300 ml Essigsäureethylester und 50 ml $H_2O$ gegossen, die organische Phase abgetrennt und die wässrige Phase nochmals mit 100 ml Essigsäureethylester nachextrahiert. Die vereinigten organischen Phasen werden über $Na_2SO_4$ getrocknet und im Vakuum bis auf ca. 50 ml Volumen eingeengt. Nach Verdünnen mit 200 ml Methylenchlorid wird mit $Na_2SO_4$ getrocknet und im Vakuum vollständig eingedampft. Der Rückstand wird an 80 g Kieselgel mit Toluol/Essigsäureethylester 3:2 chromatographiert wobei nach Kristallisation aus Ether/Hexan 3:7 die Titelverbindung erhalten wird F. 141-141,5°; Rf-Wert 0.20 (Merck Fertigplatten, Toluol/Essigsäureethylester 1:1).

Beispiel 3: (5R,6S,1'R)-2-Aminomethyl-6-(1'-hydroxyethyl)-2-penem-3-carbonsäure

Eine Lösung von 313 mg (0,85 mmol) (5R,6S,1'R)-2-Allyloxycarbonylaminomethyl-6-(1'-hydroxyethyl)-2-penem-3-carbonsäureallylester und 174,6 mg (1,24 mmol) Dimedon in 7,5 ml Tetrahydrofuran wird während 5 Minuten mit Argon gespült und unter Argon-Atmosphäre bei Raumtemperatur mit 27 mg (0,023 mmol) Tetrakistriphenylphosphin-palladium versetzt. Nach ca. 5 Minuten beginnt ein Niederschlag auszufallen, der nach weiteren 2 Stunden Rühren abfiltriert, mit ca. 10 ml Tetrahydrofuran gewaschen und anschliessend in 4 ml $H_2O$ gelöst wird. Zur beigen Lösung wird eine Mikrospatelspitze Aktivkohle und 2 Mikrospatelspitzen Tonsil gegeben, 5 Minuten gut gerührt und über Hyflo klar filtriert. Am Hochvakuum wird eingeengt und der erhaltene dicke Kristallbrei mit 3 ml kaltem Ethanol versetzt und abgenutscht. Die farblosen Kristalle werden mit 2 ml Tetrahydrofuran gewaschen und am Hochvakuum getrocknet. Man erhält die Titelverbindung vom Rf-Wert 0,50 (DC:$H_2O$, OPTI $UPC_{12}$); $[\alpha]_D^{20}$ = +176° (c = 0,5, $H_2O$).

Beispiel 4: (3S,4R,1'R)-3-(1'-Hydroxyethyl)-4-(N-cinnamyloxycarbonylglycylthio)-2-azetidinon

a) Eine Lösung von 301,8 mg (1,2 mmol) N-Cinnamyloxycarbonylthioglycin in 1,2 ml 1N wässriger NaOH wird mit 0,1N wässriger HCl auf pH 8 gestellt und zu 235 mg (1 mmol) (3R,4R,1'R)-3-(1'-Hydroxyethyl)-4-benzoyloxy-2-azetidinon in 2 ml Acetonitril bei 25° gegeben. Nach Zugabe von 0,1 ml 0,1N wässriger Natronlauge wird 25 Minuten bei 25-26° gerührt.

Zur Aufarbeitung werden 20 ml Essigsäureethylester und 10 ml Sole mit dem Reaktionsgemisch geschüttelt. Die organische Phase wird mit 5 ml 5%iger wässriger $NaHCO_3$-Lösung und zweimal mit 5 ml Sole gewaschen, über Natriumsulfat getrocknet und am Rotationsverdampfer vom Lösungsmittel befreit. Der Rückstand wird in 20 ml Toluol/Ethylacetat 2:3 gelöst, mit 1 g Kieselgel 15 Minuten verrührt und über eine Glasfritte G 2 filtriert. Man erhält nach dem Einengen die Titelverbindung als amorphes Pulver. Aus Methylenchlorid/Diisopropylether ca. 1:1 erhält man die kristallene Titelverbindung vom F. 100-101°.

Die gleiche Verbindung kann auch wie folgt erhalten werden:

b) Analog dem Verfahren a) ausgehend von 173 mg (1 mmol) (3R,4R,1'R)-3-(1'-Hydroxyethyl)-4-acetoxy-2-azetidinon und chromatographischer Reinigung des Rohprodukts, (Kieselgel, Toluol/Ethylacetat 2:3).

c) Analog dem Verfahren a) ausgehend von 235 mg (1 mmol) (3S,4R,1'R)-3-(1'-Hydroxyethyl)-4-tert.-butylsulfonyl-2-azetidinon und chromatographischer Reinigung des Rohprodukts, (Kieselgel, Toluol/Ethylacetat 2:3).

Das Ausgangsmaterial N-Cinnamyloxycarbonyl-thioglycin kann wie folgt hergesellt werden:

Zu einer Lösung von 78 ml einer 20%igen Phosgen-Lösung in Toluol wird bei 0° innert 15 Minuten eine Lösung von 13,4 g (100 mmol) Zimtalkohol in 25 ml Toluol getropft. Die rotbraune Emulsion wird unter Durchleiten von Stickstoff auf Raumtemperatur erwärmt und 2 Stunden weiter gerührt. Das Toluol wird am Vakuum (0,1 Torr) abgezogen und der Rückstand bei 0° zu einer Lösung von 3,75 g (50 mmol) Glycin, 12 ml Wasser und 6,07 ml 30%ige wässrige NaOH gegeben. Anschliessend werden bei 5 bis 15° weitere 6,07 ml 30%ige wässrige Natronlauge innert 10 Minuten zugetropft. Nach 2 Stunden rühren (pH 12) wird das Reaktionsgemisch auf 70 ml Essigsäureethylester und 70 ml Wasser gegossen. Die wässrige Phase wird abgetrennt, mit 20 ml Essigsäureethylester gewaschen, mit 2N wässriger Salzsäure auf pH 1-2 gestellt und das ausgefallene N-Cinnamyloxycarbonylglycin mit total 120 ml Essigsäureethylester extrahiert. Die vereinigten organischen Phasen werden einmal mit 10 ml Sole gewaschen, über $Na_2SO_4$ getrocknet und am Rotationsverdampfer vom Lösungsmittel befreit. Die rohe Titelverbindung wird aus 50 ml Essigsäureethylester und 25 ml Hexan kristallisiert; F. 129-130°.

Zu einer Suspension von 7,81 g (33,2 mmol) N-Cinnamyloxycarbonylglycin in 66 ml Essigsäureethylester werden bei -10 bis 15° 4,76 ml (36,4 mmol) Chlorameisensäureisobutylester gegeben und anschliessend bei der gleichen Temperatur 4 ml (36,3 mmol) N-Methyl-morpholin zugetropft. Das Reaktionsgemisch wird 1 Stunde bei -10 bis -15° weiter gerührt. Unter gleichzeitigem Einleiten von gasförmigem Schwefelwasserstoff werden innert 30 Minuten weitere 4 ml (36,3 mmol) N-Methyl-morpholin zugetropft. Nach beendigtem $H_2S$-Einleiten wird unter Rühren während 1 Stunde bei -10 bis -15° ein schwacher Stickstoffstrom eingeleitet. Nach Zutropfen von 23,2 ml 2N wässrige HCl, wobei die Temperatur auf ~0° steigt, und weiteren 30 Minuten Stickstoffspülung wird das Reaktionsgemisch auf 300 ml Essigsäureethylester gegossen, die wässrige Phase (pH 1-2) abgetrennt und einmal mit Essigsäureethylester nachextrahiert. Die vereinigten organischen Phasen werden zweimal mit 20 ml Sole gewaschen, über $Na_2SO_4$ getrocknet und vom Lösungsmittel am Rotationsverdampfer befreit. Umkristallisation des Rohproduktes aus Ether/Hexan bei 0° ergibt die analytisch reine Titelverbindung; F. 67-69°.

Beispiel 5: (5R,6S,1'R)-6-(1'-Hydroxyethyl)-2-cinnamyloxycarbonylaminomethylpenem-3-carbonsäureallylester

Eine Lösung von 310 mg (0,852 mmol) (3S,4R,1'R)-3-(1'-Hydroxyethyl)-4-(N-cinnamyloxycarbonylglycylthio)-2-azetidinon (Rohprodukt vom Beispiel 4) in 3,2 ml Methylenchlorid wird unter Rühren und Feuchtigkeitsausschluss bei -10 bis -15° nacheinander mit 0,216 ml (1,76 mmol) frisch destilliertem Oxalsäureallylesterchlorid und 0,301 ml (1,75 mmol) N-Ethyl-diisopropylamin versetzt und 30 Minuten bei -10° weiter gerührt. Eine IR-Probe ($CH_2Cl_2$) zeigt die charakteristische Oxalimid-Carbonyl-Absorption bei 1820 $cm^{-1}$. Zum Aufarbeiten wird das Reaktionsgemisch mit 2 ml $CH_2Cl_2$ verdünnt und zweimal mit je 4 ml wässriger Pufferlösung gewaschen. Die wässrigen Anteile werden einmal mit $CH_2Cl_2$ nachextrahiert. Die vereinigten organischen Phasen werden mit $Na_2SO_4$ getrocknet, im Vakuum eingedampft und das Rohprodukt am Hochvakuum getrocknet. Der erhaltene harzige Rückstand enthaltend den (3S,4R,1'R)-2-[4-(N-Cinnamyloxycarbonylglycylthio)-3-(1'-allyloxyoxalyloxyethyl)-2-oxo-1-azetidinyl]-2-oxoessigsäureallylester [IR ($CH_2Cl_2$) 3440 (NH);1820

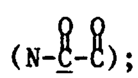

1750, 1725

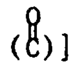

wird in 0,37 ml Dioxan gelöst und unter $N_2$-Atmosphäre bei Raumtemperatur mit 0,51 ml (2,1 mmol) Triisopropylphosphit versetzt. Nach 15 Stunden bei Raumtemperatur kann im IR kein Oxalimid mehr festgestellt werden. (Bande bei 1820 $cm^{-1}$).

Das Reaktionsgemisch wird am Vakuum (Kugelrohr, 0,1 Torr) vom überschüssigen Phosphit und vom Phosphat befreit. Das zurückbleibende rohe Phosphoran wird zur Cyclisierung in 3,3 ml Dioxan gelöst und 12 Stunden bei 100° gerührt. Das Reaktionsgemisch, enthaltend den (5R,6S,1'R)-2-Cinnamyloxycarbonylaminomethyl-6-[1'-(allyloxyoxalyloxy)ethyl]-2-penem-3-carbonsäureethylester (für analytische Zwecke wird eine Probe des rohen Cyclisierungsproduktes an Kieselgel mit Toluol/Essigsäureethylester 9:1 gereinigt; F. 87-88 °C, UV (EtOH) 315 nm, IR ($CH_2Cl_2$) 3440 (NH), 1795, 1770, 1745, 1720

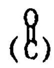

wird im Vakuum bei Raumtemperatur eingedampft und der Rückstand bei 0° in 4 ml Methanol gelöst. Anschliessend werden 2 ml 5%ige wässrige $NaHCO_3$-Lösung bei 0° zugetropft. Nach 15 Minuten werden 2 ml Wasser zugetropft und nach 30 Minuten wird die ausgefallene Titelverbindung abfiltriert und aus Methanol umkristallisiert; F. 178-180°.

Beispiel 6: (5R,6S,1'R)-2-Aminomethyl-6-(1'-hydroxyethyl)-2-penem-3-carbonsäure

Eine Lösung von 48 mg (0,108 mmol) (5R,6S,1'R)-6-(1'-Hydroxyethyl)-2-cinnamyloxycarbonylaminomethyl-2-penem-3-carbonsäureallylester und 22,3 mg (0,159 mmol) Dimedom in 1 ml Tetrahydrofuran wird während 5 Minuten mit Stickstoff gespült und unter Stickstoff-Atmosphäre bei Raumtemperatur mit 1,6 mg Tetrakistriphenylphosphin-palladium versetzt. Nach ca. 30 Minuten beginnt die Titelverbindung auszufallen. Nach weiteren 2,5 Stunden Rühren wird das Produkt abfiltriert, mit Tetrahydrofuran gut gewaschen und am Hochvakuum getrocknet.

Man erhält die Titelverbindung vom Rf 0,50 (DC $H_2O$, OPTI $UPC_{12}$); UV($H_2O$) 308 nm.

**Patentansprüche**

**Patentansprüche für folgende Vertragsstaaten : BE, CH, DE, FR, GB, IT, LI, LU, NL, SE**

**1.** Verfahren zur Herstellung von Verbindungen der Formel

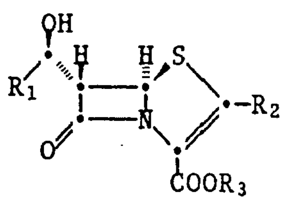

(I),

oder Salzen davon, dadurch gekennzeichnet, dass man

a) eine Verbindung der Formel

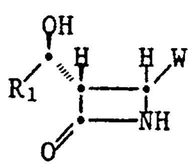

(II)

mit einer den Thiocarbonsäurerest der Formel

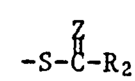

(III)

einführenden Verbindung behandelt,

b) eine erhältliche Verbindung der Formel

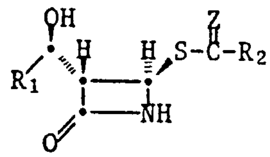

(IV)

mit einem den Acylrest eines Oxalsäurehalbesters der Formel

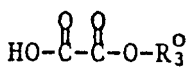

(V)

einführenden Acylierungsmittel behandelt,
c) eine erhältliche Verbindung der Formel

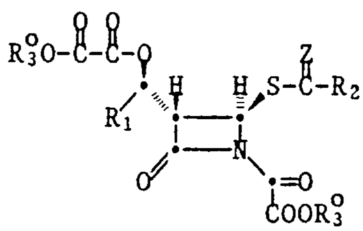

(VII)

mit einer organischen Verbindung des dreiwertigen Phosphors behandelt, und
d) in einer erhältlichen Verbindung der Formel

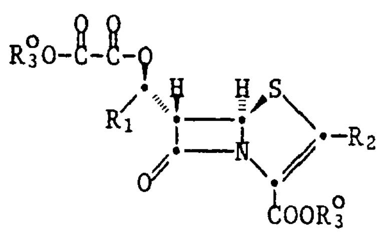

(VIII),

die Oxalylestergruppe -CO-COO-$R_3^o$ und gewünschtenfalls die Schutzgruppe $R_3^o$ und/oder eine gegebenenfalls im Rest $R_2$ vorhandene Schutzgruppe $R_2^o$ in beliebiger Reihenfolge abspaltet und durch Wasserstoff ersetzt, und gewünschtenfalls eine erhältliche Verbindung der Formel I mit salzbildender Gruppe in ein Salz oder ein erhältliches Salz in die freie Verbindung überführt, in welchen Verbindungen $R_1$ Wasserstoff oder Niederalkyl, $R_2$ ein organischer Rest, der eine funktionelle Gruppe tragen kann, die gegebenenfalls durch eine übliche Schutzgruppe $R_2^o$ geschützt sein kann, $R_3$ Wasserstoff oder eine übliche Carboxylschutzgruppe $R_3^o$ , W eine gegen einen Thiocarbonsäurerest der Formel III austauschbare Gruppe und Z Sauerstoff oder Schwefel darstellen, wobei $R_1$, $R_2$ und $R_3$ in der Penemchemie bekannte Gruppen sind.

**2.** Verfahren gemäss Anspruch 1, dadurch gekennzeichnet, dass in den Ausgangsverbindungen und Zwischenprodukten $R_1$ Wasserstoff oder Methyl darstellt, W einen Acyloxyrest, einen Sulfonylrest $-SO_2-R_o$, worin $R_o$ ein organischer Rest ist, einen Halogenrest oder Azido bedeutet, Z Sauerstoff oder Schwefel ist, $R_2$ ein organischer Rest mit bis zu 18 Kohlenstoffatomen, der bis zu 5 Stickstoffatome, und/oder bis zu 5 Sauerstoffatome, und/oder bis zu 3 Schwefelatome enthalten kann, und der sowohl über eines seiner Kohlenstoffatome als auch über ein Stickstoff-, Sauerstoff- oder Schwefelatom mit dem Penemring verbunden sein kann, ist, und $R_3^o$ eine Carboxylschutzgruppe bedeutet.

**3.** Verfahren gemäss Anspruch 1, dadurch gekennzeichnet, dass in den Ausgangsverbindungen und Zwischenprodukten $R_1$ Wasserstoff oder Methyl, W Niederalkanoyloxy, gegebenenfalls durch Nitro substituiertes Benzoyloxy oder Phenylniederalkanoyloxy, eine Gruppe $-SO_2-R_o$,worin $R_o$ gegebenenfalls durch Hydroxy substituiertes Niederalkyl, Benzyl oder gegebenenfalls z.B. durch Niederalkyl oder Halogen substituiertes Phenyl ist, oder Halogen, Z Sauerstoff oder Schwefel, $R_2$ gegebenenfalls substituiertes Niederaliphatyl, Niederaliphatyloxy, Niederaliphatylthio, Cycloaliphatyl, Cycloaliphatyloxy, Cycloaliphatylthio, Aryl, Aryloxy, Arylthio, Arylniederaliphatyl, Arylniederaliphatyloxy, Arylniederaliphatylthio, Heterocyclyl, Heterocyclyloxy, Heterocyclylthio, Heterocyclylniederaliphatyl, Heterocyclylniederaliphatyloxy, Heterocyclylniederaliphatylthio, Heterocyclyloxyniederaliphatyl oder Heterocyclylthioniederaliphatyl, wobei Niederaliphatyl, Cycloaliphatyl und Heterocyclyl gesättigt oder ungesättigt sein können und $R_3^o$ Niederalkyl, Arylmethyl, Heteroarylmethyl, Niederalkanoylmethyl, Aroylmethyl, Halogenniederalkyl, Niederalkenyl, 2-Niederalkylsulfonyl-, 2-Cyano-, 2-Triniederalkylsilyl- oder 2-Triphenylsilylethyl bedeuten.

**4.** Verfahren gemäss Anspruch 1, dadurch gekennzeichnet, dass in den Ausgangsverbindungen und Zwischenprodukten $R_3^o$ Allyl bedeutet.

**5.** Verfahren gemäss Anspruch 1, dadurch gekennzeichnet, dass in den Ausgangsverbindungen und Zwischenprodukten $R_1$ Wasserstoff oder Methyl, W Benzoyloxy, Z Sauerstoff oder Schwefel, $R_2$ Aminomethyl, 2-Aminoprop-1-yl, Carbamoyloxymethyl, 1-Methyltetrazol-5-ylthiomethyl, 2-(1-Tetrazolyl)-propyl, 2-Oxo-5-tetrahydrofurylmethyl, 2-Oxo-5-pyrrolidylmethyl, 2-Aminoethylthio, 2-Formamidinoethylthio, 2-Carbamoyloxyethylthio, 1-Imidazolyl, 4,5-Dimethyl-1-imidazolyl, 4-Methyl-5-thiazolyl, 5-Methyl-4-thiazolyl und 2-Amino-4-methyl-5-thiazolyl, und ihre geschützten Derivate und $R_3^o$ Allyl bedeuten.

**6.** Verfahren gemäss Anspruch 1, dadurch gekennzeichnet, dass in den Ausgangsverbindungen und Zwischenprodukten $R_2^o$ Allyloxycarbonyl bedeutet.

**7.** Verfahren gemäss Anspruch 1, dadurch gekennzeichnet, dass man (3R,4R,1'R)-3-(1'-Hydroxyethyl)-4-benzoyloxy-2-azetidinon oder (3S,4R,1'R))-3-(1'-Hydroxyethyl)-4-tert.-butylsulfonyl-2-azetidinonmit N-Allyloxycarbonylthioglycin umsetzt, das erhältliche (3S,4R,1'R)-3-(1'-Hydroxyethyl)-4-(N-allyloxycarbonylglycylthio)-2-azetidinon in Gegenwart von N-Ethyl-diisopropylamin mit Oxalsäureallylesterchlorid behandelt, und aus den erhältlichen (5R,6S,1'R)-2-Allyoxycarbonylaminomethyl-6-[1'-(allyloxyoxalyloxy)ethyl]-2-penem-3-carbonsäureallylester die Schutzgruppen an der 1'-Hydroxygruppe und an der 3-Carboxylgruppe abspaltet und die (5R,6S,1'R)-2-Aminomethyl-6-(1'-hydroxyethyl)-2-penem-3-carbonsäure isoliert.

**8.** Verfahren gemäss Anspruch 1, dadurch gekennzeichnet, dass in den Ausgangsverbindungen und Zwischenprodukten $R_2^o$ Cinnamyloxycarbonyl bedeutet.

**9.** Verfahren gemäss Anspruch 1, dadurch gekennzeichnet, dass man (3R,4R,1'R)-3-(1'-Hydroxyethyl)-4-benzoyloxy-2-azetidinon, (3R,4R,1'R)-3-(1'-Hydroxyethyl)-4-acetoxy-2-azetidinon oder (3S,4R,1'R))-3-(1'-Hydroxyethyl)-4-tert.-butylsulfonyl-2-azetidinonmit N-Cinnamyloxycarbonylthioglycin umsetzt, das erhältliche (3S,4R,1'R)-3-(1'-Hydroxyethyl)-4-(N-Cinnamyloxycarbonylglycylthio)-2-azetidinon in Gegenwart von N-Ethyl-diisopropylamin mit Oxalsäureallylesterchlorid behandelt, und aus den erhältlichen (5R,6S,1'R)-2-Allyoxycarbonylaminomethyl-6-[1'-(allyloxyoxalyloxy)ethyl]-2-penem-3-carbonsäureallylester die Schutzgruppen an der 1'-Hydroxygruppe und an der 3-Carboxylgruppe abspaltet und die (5R,6S,1'R)-2-Aminomethyl-6-(1'-hydroxyethyl)-2-penem-3-carbonsäure isoliert.

**10.** Verbindungen der Formel I gemäss Anspruch 1, worin $R_2$ Cinnamyloxycarbonylaminomethyl ist und $R_1$ und $R_3$ die angegebenen Bedeutungen haben.

**11.** (5R,6S,1'R)-(1'-Hydroxyethyl)-2-cinnamyloxycarbonylaminomethylpenem-3-carbonsäureallylester nach Anspruch 10.

**12.** Verbindungen der Formel IV gemäss Anspruch 1, worin $R_2$ Cinnamyloxycarbonylaminomethyl ist und $R_1$ und Z die angegebenen Bedeutungen haben.

**13.** (3S,4R,1'R)-3-(1'-Hydroxyethyl)-4-(N-cinnamyloxycarbonylglycylthio)-2-azetidinon nach Anspruch 12.

**14.** (3S,4R,1'R)-2-[4-(N-Cinnamyloxycarbonylglycylthio)-3-(1'-allyloxyoxalyloxyethyl)-2-oxo-1-azetidinyl]-2-oxoessigsäureallylester.

**15.** (5R,6S,1'R)-2-Cinnamyloxycarbonylaminomethyl-6-[1'-(allyloxyoxalyloxy)ethyl]-2-penem-3-carbonsäureallylester.

**Patentansprüche für folgenden Vertragsstaat : AT**

**1.** Verfahren zur Herstellung von Verbindungen der Formel

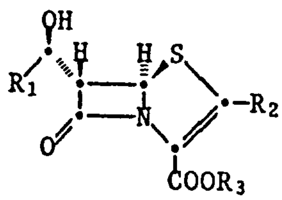

(I),

oder Salzen davon, dadurch gekennzeichnet, dass man

a) eine Verbindung der Formel

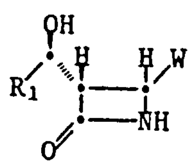

(II)

mit einer den Thiocarbonsäurerest der Formel

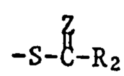

(III)

einführenden Verbindung behandelt,

b) eine erhältliche Verbindung der Formel

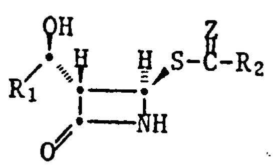

(IV)

mit einem den Acylrest eines Oxalsäurehalbesters der Formel

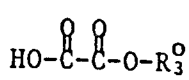

(V)

einführenden Acylierungsmittel behandelt,

c) eine erhältliche Verbindung der Formel

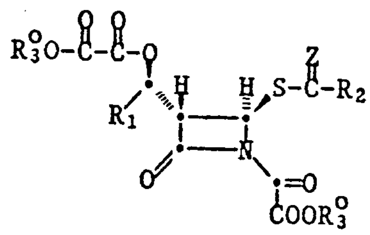

(VII)

mit einer organischen Verbindung des dreiwertigen Phosphors behandelt, und
d) in einer erhältlichen Verbindung der Formel

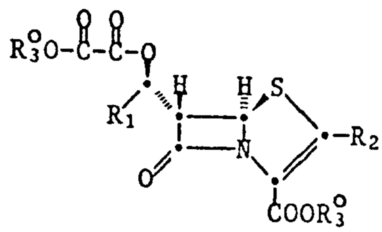

(VIII),

die Oxalylestergruppe -CO-COO-$R_3^o$ und gewünschtenfalls die Schutzgruppe $R_3^o$ und/oder eine gegebenenfalls im Rest $R_2$ vorhandene Schutzgruppe $R_2^o$ in beliebiger Reihenfolge abspaltet und durch Wasserstoff ersetzt, und gewünschtenfalls eine erhältliche Verbindung der Formel I mit salzbildender Gruppe in ein Salz oder ein erhältliches Salz in die freie Verbindung überführt, in welchen Verbindungen $R_1$ Wasserstoff oder Niederalkyl, $R_2$ ein organischer Rest, der eine funktionelle Gruppe tragen kann, die gegebenenfalls durch eine übliche Schutzgruppe $R_2^o$ geschützt sein kann, $R_3$ Wasserstoff oder eine übliche Carboxylschutzgruppe $R_3^o$ , W eine gegen einen Thiocarbonsäurerest der Formel III austauschbare Gruppe und Z Sauerstoff oder Schwefel darstellen, wobei $R_1$, $R_2$ und $R_3$ in der Penemchemie bekannte Gruppen sind.

**2.** Verfahren gemäss Anspruch 1, dadurch gekennzeichnet, dass in den Ausgangsverbindungen und Zwischenprodukten $R_1$ Wasserstoff oder Methyl darstellt, W einen Acyloxyrest, einen Sulfonylrest -$SO_2$-$R_o$, worin $R_o$ ein organischer Rest ist, einen Halogenrest oder Azido bedeutet, Z Sauerstoff oder Schwefel ist, $R_2$ ein organischer Rest mit bis zu 18 lenstoffatomen, der bis zu 5 Stickstoffatome, und/oder bis zu 5 Sauerstoffatome, und/oder bis zu 3 Schwefelatome enthalten kann, und der sowohl über eines seiner Kohlenstoffatome als auch über ein Stickstoff-, Sauerstoff- oder Schwefelatom mit dem Penemring verbunden sein kann, ist, und $R_3^o$ eine Carboxylschutzgruppe bedeutet.

**3.** Verfahren gemäss Anspruch 1, dadurch gekennzeichnet, dass in den Ausgangsverbindungen und Zwischenprodukten $R_1$ Wasserstoff oder Methyl, W Niederalkanoyloxy, gegebenenfalls durch Nitro substituiertes Benzoyloxy oder Phenylniederalkanoyloxy, eine Gruppe -$SO_2$-$R_o$,worin $R_o$ gegebenenfalls durch Hydroxy substituiertes Niederalkyl, Benzyl oder gegebenenfalls z.B. durch Niederalkyl oder Halogen substituiertes Phenyl ist, oder Halogen, Z Sauerstoff oder Schwefel, $R_2$ gegebenenfalls substituiertes Niederaliphatyl, Niederaliphatyloxy, Niederaliphatylthio, Cycloaliphatyl, Cycloaliphatyloxy, Cycloaliphatylthio, Aryl, Aryloxy, Arylthio, Arylniederaliphatyl, Arylniederaliphatyloxy, Arylniederaliphatylthio, Heterocyclyl, Heterocyclyloxy, Heterocyclylthio, Heterocyclylniederaliphatyl, Heterocyclylniederaliphatyloxy, Heterocyclylniederaliphatylthio, Heterocyclyloxyniederaliphaty oder Heterocyclylthioniederaliphatyl, wobei Niederaliphatyl, Cycloaliphatyl und Heterocyclyl gesättigt oder ungesättigt sein können und $R_3^o$ Niederalkyl, Arylmethyl, Heteroarylmethyl, Niederalkanoylmethyl, Aroylmethyl, Halogenniederalkyl, Niederalkenyl, 2-Niederalkylsulfonyl-, 2-Cyano-, 2-Triniederalkylsilyl- oder 2-Triphenylsilylethyl bedeuten.

**4.** Verfahren gemäss Anspruch 1, dadurch gekennzeichnet, dass in den Ausgangsverbindungen und Zwischenprodukten $R_3^o$ Allyl bedeutet.

**5.** Verfahren gemäss Anspruch 1, dadurch gekennzeichnet, dass in den Ausgangsverbindungen und Zwischenprodukten $R_1$ Wasserstoff oder Methyl, W Benzoyloxy, Z Sauerstoff oder Schwefel, $R_2$ Aminomethyl, 2-Aminoprop-1-yl, Carbamoyloxymethyl, 1-Methyltetrazol-5-ylthiomethyl, 2-(1-Tetrazolyl)-propyl, 2-Oxo-5-tetrahydrofurylmethyl, 2-Oxo-5-pyrrolidylmethyl, 2-Aminoethylthio, 2-Formamidinoethylthio, 2-Carbamoyloxyethylthio, 1-Imidazolyl, 4,5-Dimethyl-1-imidazolyl, 4-Methyl-5-thiazolyl, 5-Methyl-4-thiazolyl und 2-Amino-4-methyl-5-thiazolyl, und ihre geschützten Derivate und $R_3^o$ Allyl bedeuten.

**6.** Verfahren gemäss Anspruch 1, dadurch gekennzeichnet, dass in den Ausgangsverbindungen und Zwischenprodukten $R_2^o$ Allyloxycarbonyl bedeutet.

**7.** Verfahren gemäss Anspruch 1, dadurch gekennzeichnet, dass man (3R,4R,1'R)-3-(1'-Hydroxyethyl)-4-benzoyloxy-2-azetidinon oder (3S,4R,1'R))-3-(1'-Hydroxyethyl)-4-tert.-butylsulfonyl-2-azetidinonmit N-Allyloxycarbonylthioglycin umsetzt, das erhältliche (3S,4R,1'R)-3-(1'-Hydroxyethyl)-4-(N-allyloxycarbonylglycylthio)-2-azetidinon in Gegenwart von N-Ethyl-diisopropylamin mit Oxalsäureallylesterchlorid behandelt, und aus den erhältlichen (5R,6S,1'R)-2-Allyoxycarbonylaminomethyl-6-[1'-(allyloxyoxalyloxy)ethyl]-2-penem-3-carbonsäureallylester die Schutzgruppen an der 1'-Hydroxygruppe und an der 3-Carboxylgruppe abspaltet und die (5R,6S,1'R)-2-Aminomethyl-6-(1'hydroxyethyl)-2-penem-3-carbonsäure isoliert.

**8.** Verfahren gemäss Anspruch 1, dadurch gekennzeichnet, dass in den Ausgangsverbindungen und Zwischenprodukten $R_2^o$ Cinnamyloxycarbonyl bedeutet.

**9.** Verfahren gemäss Anspruch 1, dadurch gekennzeichnet, dass man (3R,4R,1'R)-3-(1'-Hydroxyethyl)-4-benzoyloxy-2-azetidinon, (3R,4R,1'R)-3-(1'-Hydroxyethyl)-4-acetoxy-2-azetidinon oder (3S,4R,1'R))-3-(1'-Hydroxyethyl)-4-tert.-butylsulfonyl-2-azetidinonmit N-Cinnamyloxycarbonylthioglycin umsetzt, das erhältliche (3S,4R,1'R)-3-(1'-Hydroxyethyl)-4-(N-Cinnamyloxycarbonylglycylthio)-2-azetidinon in Gegenwart von N-Ethyl-diisopropylamin mit Oxalsäureallylesterchlorid behandelt, und aus den erhältlichen (5R,6S,1'R)-2-Allyoxycarbonylaminomethyl-6-[1'-(allyloxyoxalyloxy)ethyl]-2-penem-3-carbonsäureallylester die Schutzgruppen an der 1'-Hydroxygruppe und an der 3-Carboxylgruppe abspaltet und die (5R,6S,1'R)-2-Aminomethyl-6-(1'-hydroxyethyl)-2-penem-3-carbonsäure isoliert.

## Claims

**Claims for the following Contracting States : BE, CH, DE, FR, GB, IT, LI, LU, NL, SE**

**1.** A process for the preparation of a compound of formula

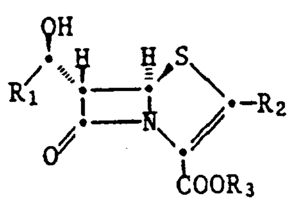

(I),

or a salt thereof, which comprises

a) treating a compound of formula

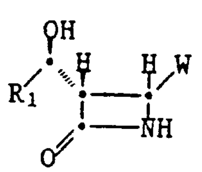

(II)

with a compound that introduces a thiocarboxylic acid radical of formula

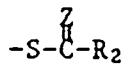

(III),

b) treating a compound which may be obtained of formula

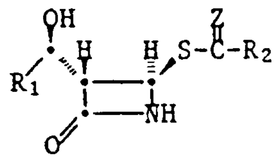

(IV)

with an acylating agent that introduces the acyl radical of an oxalic acid hemiester of formula

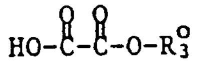

(V),

c) treating a compound which may be obtained of formula

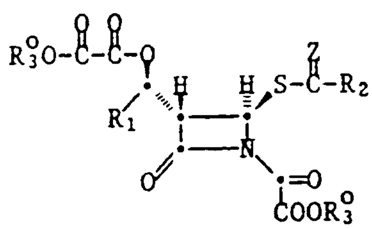

(VII)

with an organic compound of trivalent phosphorus, and
d) removing the oxalyl ester group -CO-COO-$R_3^o$ and, if desired, the protective group $R_3^o$ and/or a protective group $R_2^o$ which may be present in the radical $R_2$, in any order, from a compound which may be obtained of formula

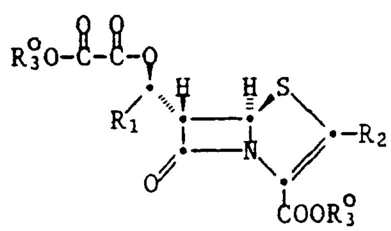

(VIII),

and replacing said group or groups with hydrogen and, if desired, converting a compound which may be obtained of formula I containing a salt-forming group into a salt or converting a salt which may be obtained into the free compound, in which compounds $R_1$ is hydrogen or lower alkyl, $R_2$ is an organic radical which may carry a functional group which may optionally be protected by a customary protective group $R_2^o$ , $R_3$ is hydrogen or a customary carboxyl-protective group $R_3^o$ , W is a group which can be replaced by a thiocarboxylic acid radical of formula III, and Z is oxygen or sulfur, $R_1$, $R_2$ and $R_3$ being groups known in penem chemistry.

**2.** A process according to claim 1, wherein in the starting compounds and intermediate products $R_1$ is hydrogen or methyl, W is an acyloxy radical, a sulfonyl radical -$SO_2$-$R_o$, in which $R_o$ is an organic radical, a halogen substituent or azido, Z is oxygen or sulfur, $R_2$ is an organic radical containing up to 18 carbon atoms, and which may contain up to 5 nitrogen atoms, and/or up to 5 oxygen atoms, and/or

up to 3 sulfur atoms, and which may be attached to the penem ring through one of its carbon atoms as well as through a nitrogen. oxygen or sulfur atom, and $R_3^o$ is a carboxyl-protective group.

**3.** A process according to claim 1, wherein in the starting compounds and intermediate products $R_1$ is hydrogen or methyl, W is lower alkanoyloxy, or benzoyloxy or phenyl-lower alkanoyloxy, each unsubstituted or substituted by nitro, or is a group $-SO_2-R_o$, in which $R_0$ is lower alkyl or hydroxy-substituted lower alkyl, benzyl, phenyl or phenyl which is substituted e.g. by lower alkyl or halogen, or is halogen, Z is oxygen or sulfur, $R_2$ is unsubstituted or substituted lower aliphatyl, lower aliphatyloxy, lower aliphatylthio, cycloaliphatyl, cycloaliphatyloxy, cycloaliphatylthio, aryl, aryloxy, arylthio, aryl-lower aliphatyl, aryl-lower aliphatyloxy, aryl-lower aliphatylthio, heterocyclyl, heterocyclyloxy, heterocyclylthio. heterocyclyl-lower aliphatyl, heterocyclyl-lower aliphatyloxy, heterocyclyl-lower aliphatylthio, heterocyclyloxy-lower aliphatyl or heterocyclylthio-lower aliphatyl, with lower aliphatyl, cycloaliphatyl and heterocyclyl being able to be saturated or unsaturated, and $R_3^o$ is lower alkyl, arylmethyl, heteroarylmethyl, lower alkanoylmethyl, aroylmethyl, halo-lower alkyl, lower alkenyl, 2-lower alkylsulfonylethyl, 2-cyanoethyl, 2-tri-lower alkylsilylethyl or 2-triphenylsilylethyl.

**4.** A process according to claim 1, wherein in the starting compounds and intermediate products $R_3^o$ is allyl.

**5.** A process according to claim 1, wherein in the starting compounds and intermediate products $R_1$ is hydrogen or methyl, W is benzoyloxy, Z is oxygen or sulfur, $R_2$ is aminomethyl, 2-amino-prop-1-yl, carbamoyloxymethyl, 1-methyltetrazol-5-ylthiomethyl, 2-(1-tetrazolyl)propyl, 2-oxo-5-tetrahydrofurylmethyl, 2-oxo-5-pyrrolidinylmethyl, 2-aminoethylthio, 2-formamidinoethylthio, 2-carbamoyloxyethylthio, 1-imidazolyl, 4,5-dimethyl-1-imidazolyl, 4-methyl-5-thiazolyl, 5-methyl-4-thiazolyl and 2-amino-4-methyl-5-thiasolyl, and the protected derivatives thereof, and $R_3^o$ is allyl.

**6.** A process according to claim 1, wherein in the starting compounds and intermediate products $R_2^o$ is allyloxycarbonyl.

**7.** A process according to claim 1, which comprises reacting (3R,4R,1'R)-3-(1'-hydroxyethyl)-4-benzoyloxy-2-azetidinone or (3S,4R,1'R)-3-(1'-hydroxyethyl)-4-tert-butylsulfonyl-2-azetidinonewith N-allyloxycarbonylthioglycine, treating the (3S,4R,1'R)-3-(1'-hydroxyethyl)-4-(N-allyloxycarbonylglycylthio)-2-azetidinone which may be obtained, in the presence of N-ethyldiisopropylamine, with allyl oxalate chloride, and removing the protective groups from the allyl (5S,6R,1'R)-2-allyloxycarbonylaminomethyl-6-[1'-(allyloxyoxalyloxy)ethyl]-2-penem-3-carboxylate which may be obtained at the 1'-hydroxyl group and at the 3-carboxyl group, and isolating the (5R,6S,1'R)-2-aminomethyl-6-(1'-hydroxyethyl)-2-penem-3-carboxylic acid.

**8.** A process according to claim 1, wherein in the starting compounds and intermediate products $R_2^o$ is cinnamyloxycarbonyl.

**9.** A process according to claim 1, which comprises reacting (3R,4R,1'R)-3-(1'-hydroxyethyl)-4-benzoyloxy-2-azetidinone, (3R,4R,1'R)-3-(1'-hydroxyethyl)-4-acetoxy-2-azetidinone or (3S,4R,1'R)-3-(1'-hydroxyethyl)-4-tert-butylsulfonyl-2-azetidinonewith N-cinnamyloxycarbonylthioglycine, treating the (3S,4R,1'R)-3-(1'-hydroxyethyl)-4-(N-cinnamyloxycarbonylglycylthio)-2-azetidinone which may be obtained, in the presence of N-ethyldiisopropylamine, with allyl oxalate chloride, and removing the protective groups from the allyl (5S,6R,1'R)-2-allyloxycarbonylaminomethyl-6-[1'-(allyloxyoxalyloxy)-ethyl]-2-penem-3-carboxylate which may be obtained at the 1'-hydroxyl group and at the 3-carboxyl group, and isolating the (5R,6S,1'R)-2-aminomethyl-6-(1'-hydroxyethyl)-2-penem-3-carboxylic acid.

**10.** A compound of formula I according to claim 1, wherein $R_2$ is cinnamyloxycarbonylaminomethyl and $R_1$ and $R_2$ have the given meanings.

**11.** Allyl (5R,6S,1'R)-3-(1'-hydroxyethyl)-2-cinnamyloxycarbonylaminomethyl-penem-3-carboxylate according to claim 10.

**12.** A compound of formula IV according to claim 1, wherein $R_2$ is cinnamyloxycarbonylaminomethyl and $R_1$ and Z have the given meanings.

**13.** (3S,4R,1'R)-3-(1'-Hydroxyethyl)-4-(N-cinnamyloxycarbonylglycylthio)-2-azetidinone according to claim 12.

**14.** Allyl (3S,4R,1'R)-2-[4-(N-cinnamyloxycarbonylglycylthio)-3-(1'-allyloxyoxalyloxyethyl)-2-oxo-1-azetidinyl]-2-oxoacetate.

**15.** Allyl (5R,6S,1'R)-2-cinnamyloxycarbonylaminomethyl-6-[1'-(allyloxyoxalyloxy)ethyl]-2-penem-3-carboxylate.

**Claims for the following Contracting State : AT**

**1.** A process for the preparation of a compound of formula

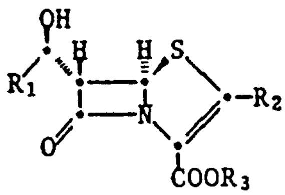

(I),

or a salt thereof, which comprises

a) treating a compound of formula

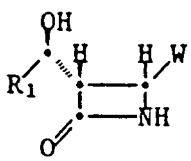

(II)

with a compound that introduces a thiocarboxylic acid radical of formula

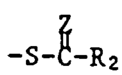

(III),

b) treating a compound which may be obtained of formula

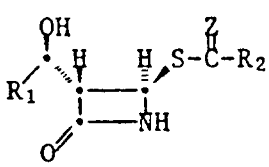

(IV)

with an acylating agent that introduces the acyl radical of an oxalic acid hemiester of formula

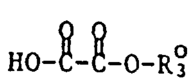

(V),

c) treating a compound which may be obtained of formula

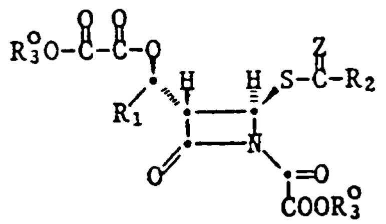

(VII)

with an organic compound of trivalent phosphorus, and

d) removing the oxalyl ester group -CO-COO-$R_3^o$ and, if desired, the protective group $R_3^o$ and/or a protective group $R_2^o$ which may be present in the radical $R_2$, in any order, from a compound which may be obtained of formula

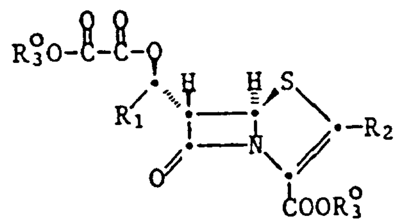

(VIII),

and replacing said group or groups with hydrogen and, if desired, converting a compound which may be obtained of formula I containing a salt-forming group into a salt or converting a salt which may be obtained into the free compound, in which compounds $R_1$ is hydrogen or lower alkyl, $R_2$ is an organic radical which may carry a functional group which may optionally be protected by a customary protective group $R_2^o$ , $R_3$ is hydrogen or a customary carboxyl-protective group $R_3^o$ , W is a group which can be replaced by a thiocarboxylic acid radical of formula III, and Z is oxygen or sulfur, $R_1$, $R_2$ and $R_3$ being groups known in penem chemistry.

**2.** A process according to claim 1, wherein in the starting compounds and intermediate products $R_1$ is hydrogen or methyl, W is an acyloxy radical, a sulfonyl radical -$SO_2$-$R_o$, in which $R_o$ is an organic radical, a halogen substituent or azido, Z is oxygen or sulfur, $R_2$ is an organic radical containing up to 18 carbon atoms, and which may contain up to 5 nitrogen atoms, and/or up to 5 oxygen atoms, and/or up to 3 sulfur atoms, and which may be attached to the penem ring through one of its carbon atoms as well as through a nitrogen, oxygen or sulfur atom, and $R_3^o$ is a carboxyl-protective group.

**3.** A process according to claim 1, wherein in the starting compounds and intermediate products $R_1$ is hydrogen or methyl, W is lower alkanoyloxy, or benzoyloxy or phenyl-lower alkanoyloxy, each unsubstituted or substituted by nitro, or is a group -$SO_2$-$R_o$, in which $R_o$ is lower alkyl or hydroxy-substituted lower alkyl, benzyl, phenyl or phenyl which is substituted e.g. by lower alkyl or halogen, or is halogen, Z is oxygen or sulfur, $R_2$ is unsubstituted or substituted lower aliphatyl, lower aliphatyloxy, lower aliphatylthio, cycloaliphatyl, cycloaliphatyloxy, cycloaliphatylthio, aryl, aryloxy, arylthio, aryl-lower aliphatyl, aryl-lower aliphatyloxy, aryl-lower aliphatylthio, heterocyclyl, heterocyclyloxy, heterocyclylthio, heterocyclyl-lower aliphatyl, heterocyclyl-lower aliphatyloxy, heterocyclyl-lower aliphatylthio, heterocyclyloxy-lower aliphatyl or heterocyclylthio-lower aliphatyl, with lower aliphatyl, cycloaliphatyl and heterocyclyl being able to be saturated or unsaturated, and $R_3^o$ is lower alkyl, arylmethyl, heteroarylmethyl, lower alkanoylmethyl, aroylmethyl, halo-lower alkyl, lower alkenyl, 2-lower alkylsulfonylethyl, 2-cyanoethyl, 2-tri-lower alkylsilylethyl or 2-triphenylsilylethyl.

**4.** A process according to claim 1, wherein in the starting compounds and intermediate products $R_3^o$ is allyl.

5. A process according to claim 1, wherein in the starting compounds and intermediate products $R_1$ is hydrogen or methyl, W is benzoyloxy, Z is oxygen or sulfur, $R_2$ is aminomethyl, 2-amino-prop-1-yl, carbamoyloxymethyl, 1-methyltetrasol-5-ylthiomethyl, 2-(1-tetrazolyl)propyl, 2-oxo-5-tetrahydrofurylmethyl, 2-oxo-5-pyrrolidinylmethyl, 2-aminoethylthio, 2-formamidinoethylthio, 2-carbamoyloxyethylthio, 1-imidazolyl, 4,5-dimethyl-1-imidazolyl, 4-methyl-5-thiazolyl, 5-methyl-4-thiazolyl and 2-amino-4-methyl-5-thiazolyl, and the protected derivatives thereof, and $R_3^o$ is allyl.

6. A process according to claim 1, wherein in the starting compounds and intermediate products $R_2^o$ is allyloxycarbonyl.

7. A process according to claim 1, which comprises reacting (3R,4R,1'R)-3-(1'-hydroxyethyl)-4-benzoyloxy-2-azetidinone or (3S,4R,1'R)-3-(1'-hydroxyethyl)-4-tert-butylsulfonyl-2-azetidinonewith N-allyloxycarbonylthioglycine, treating the (3S,4R,1'R)-3-(1'-hydroxyethyl)-4-(N-allyloxycarbonylglycylthio)-2-azetidinone which may be obtained, in the presence of N-ethyldiisopropylamine, with allyl oxalate chloride, and removing the protective groups from the allyl (5S,6R,1'R)-2-allyloxycarbonylaminomethyl-6-[1'-(allyloxyoxalyloxy)ethyl]-2-penem-3-carboxylate which may be obtained at the 1'-hydroxyl group and at the 3-carboxyl group, and isolating the (5R,6S,1'R)-2-aminomethyl-6-(1'-hydroxyethyl)-2-penem-3-carboxylic acid.

8. A process according to claim 1, wherein in the starting compounds and intermediate products $R_2^o$ is cinnamyloxycarbonyl.

9. A process according to claim 1, which comprises reacting (3R,4R,1'R)-3-(1'-hydroxyethyl)-4-benzoyloxy-2-azetidinone, (3R,4R,1'R)-3-(1'-hydroxyethyl)-4-acetoxy-2-azetidinone or (3S,4R,1'R)-3-(1'-hydroxyethyl)-4-tert-butylsulfonyl-2-azetidinonewith N-cinnamyloxycarbonylthioglycine, treating the (3S,4R,1'R)-3-(1'-hydroxyethyl)-4-(N-cinnamyloxycarbonylglycylthio)-2-azetidinone which may be obtained, in the presence of N-ethyldiisopropylamine, with allyl oxalate chloride, and removing the protective groups from the allyl (5S,6R,1'R)-2-allyloxycarbonylaminomethyl-6-[1'-(allyloxyoxalyloxy)-ethyl]-2-penem-3-carboxylate which may be obtained at the 1'-hydroxyl group and at the 3-carboxyl group, and isolating the (5R,6S,1'R)-2-aminomethyl-6-(1'-hydroxyethyl)-2-penem-3-carboxylic acid.

**Revendications**
**Revendications pour les Etats contractants suivants : BE, CH, DE, FR, GB, IT, LI, LU, NL, SE**

1. Procédé de préparation de composés de formule

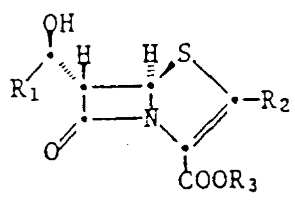

(I),

ou de leurs sels, caractérisé en ce que :
a) on traite un composé de formule

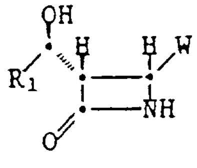

(II)

par un composé introduisant le radical d'acide thiocarboxylique de formule

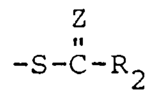

(III)

b) on traite le composé ainsi obtenu, de formule

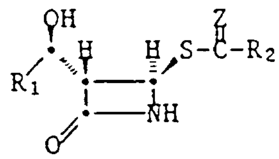

(IV)

par un agent acylant introduisant le radical acyle d'un hémiester oxalique de formule

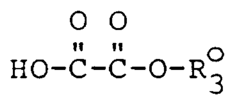

(V)

c) on traite le composé obtenu répondant à la formule

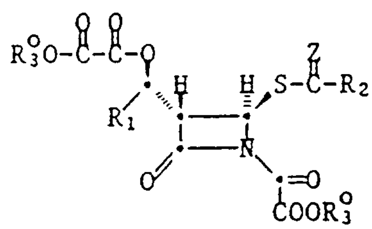

(VII)

par un dérivé organique du phosphore trivalent, et

d) dans le composé obtenu, répondant à la formule

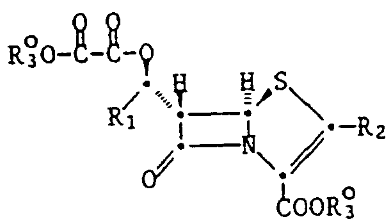

(VIII),

on élimine dans un ordre quelconque le groupe ester oxalique -CO-COO-$R_3^o$ et si on le désire le groupe protecteur $R_3^o$ et/ou un groupe protecteur $R_2^o$ éventuellement présent dans le groupe $R_2$ et on les remplace par l'hydrogène, et si on le désire, on convertit un composé obtenu répondant à la formule I et contenant un groupe salifiable en un sel ou un sel obtenu en le composé libre, les symboles des formules ci-dessus ayant les significations suivantes : $R_1$ représente l'hydrogène ou un groupe alkyle inférieur, $R_2$ représente un radical organique qui peut porter un groupe fonctionnel qui peut éventuellement être protégé par un groupe protecteur usuel $R_2^o$ , $R_3$ représente l'hydrogène ou un groupe protecteur usuel du groupe carboxyle $R_3^o$ , W représente un groupe échangeable contre le radical d'acide thiocarboxylique de formule III et Z représente l'oxygène ou le soufre, $R_1$, $R_2$ et $R_3$ représentant des groupes connus dans la chimie des pénèmes.

**2.** Procédé selon revendication 1, caractérisé en ce que, dans les composés de départ et produits intermédiaires, $R_1$ représente l'hydrogène ou un groupe méthyle, W représente un radical acyloxy, un

groupe sulfonyle-$SO_2$-$R_o$ dans lequel $R_o$ représente un radical organique, un radical halogéné ou un groupe azido, Z représente l'oxygène ou le soufre, $R_2$ un radical organique contenant jusqu'à 18 atomes de carbone et qui peut contenir jusqu'à 5 atomes d'azote et/ou jusqu'à 5 atomes d'oxygène et/ou jusqu'à 3 atomes de soufre, et qui peut être relié au cycle pénème aussi bien par l'un de ses atomes de carbone que par un atome d'azote, d'oxygène ou de soufre, et $R_3^o$ représente un groupe protecteur du groupe carboxyle.

3. Procédé selon la revendication 1, caractérisé en ce que, dans les composés de départ et produits intermédiaires, $R_1$ représente l'hydrogène ou un groupe méthyle, W représente un groupe alcanoyloxy inférieur, un groupe benzoyloxy ou phényl-alcanoyloxy inférieur éventuellement substitué par des groupes nitro, un groupe -$SO_2$-$R_o$ dans lequel $R_o$ représente un groupe alkyle inférieur éventuellement substitué par un groupe hydroxy, un groupe benzyle ou un groupe phényle éventuellement substitué par exemple par un groupe alkyle inférieur ou un halogène, ou un halogène, Z représente l'oxygène ou le soufre, $R_2$ représente un groupe aliphatyle inférieur, aliphatyloxy inférieur, aliphatylthio inférieur, cycloaliphatyle, cycloaliphatyloxy, cycloaliphatylthio, aryle, aryloxy, arylthio, aryl-aliphatyle inférieur, aryl-aliphatyloxy inférieur, aryl-aliphatylthio inférieur, hétérocyclyle, hétérocyclyloxy, hétérocyclylthio, hétérocyclyl-aliphatyle inférieur, hétérocyclyl-aliphatyloxy inférieur, hétérocyclyl-aliphatylthio inférieur, hétérocyclyloxy-aliphatyle inférieur ou hétérocyclylthio-aliphatyle inférieur éventuellement substitué, les groupes aliphatyle inférieurs, cycloaliphatyle et hétérocyclyle pouvant être saturés ou insaturés, et $R_3^o$ représente un groupe alkyle inférieur, arylméthyle, hétéroarylméthyle, (alcanoyle inférieur)-méthyle, aroylméthyle, halogénoalkyle inférieur, alcényle inférieur, 2-alkylsulfonyle inférieur, 2-cyano-, 2-tri-(alkyle inférieur)-silyl- ou 2-triphénylsilyléthyle.

4. Procédé selon revendication 1, caractérisé en ce que, dans les composés de départ et les produits intermédiaires, $R_3^o$ représente un groupe allyle.

5. Procédé selon revendication 1, caractérisé en ce que, dans les composés de départ et produits intermédiaires, $R_1$ représente l'hydrogène ou un groupe méthyle, W un groupe benzoyloxy, Z l'oxygène ou le soufre, $R_2$ un groupe aminométhyle, 2-amino-propa-1-yle, carbamoyloxyméthyle, 1-méthyltétrazole-5-ylthiométhyle, 2-(1-tétrazolyl)-propyle, 2-oxo-5-tétrahydrofurylméthyle, 2-oxo-5-pyrrolidylméthyle, 2-aminoéthylthio, 2-formamidinoéthylthio, 2-carbamoyloxyéthylthio, 1-imidazolyle, 4,5-diméthyl-1-imidazolyle, 4-méthyl-5-thiazolyle, 5-méthyl-4-thiazolyle et 2-amino-4-méthyl-5-thiazolyle ou leurs dérivés protégés et $R_3^o$ représente un groupe allyle.

6. Procédé selon revendication 1, caractérisé en ce que, dans les composés de départ et produits intermédiaires, $R_2^o$ représente un groupe allyloxycarbonyle.

7. Procédé selon revendication 1, caractérisé en ce que l'on fait réagir la (3R,4R,1'R)-3-(1'-hydroxyéthyl)-4-benzoyloxy-2-azétidinone ou la (3S,4R,1'R)-3-(1'-hydroxyéthyl)-4-tert-butylsulfonyl-2-azétidinone avec la N-allyloxycarbonylthioglycine, ce qui donne la (3S,4R,1'R)-3-(1'-hydroxyéthyl)-4-(N-allyloxycarbonylthioglycylthio)-2-azétidinone qu'on traite en présence de la N-éthyldiisopropylamine par le chlorure-ester allylique de l'acide oxalique, ce qui donne des (5R,6S, 1'R)-2-allyloxycarbonylaminométhyl-6-[1'-(allyloxyoxalyloxy-éthyl]-2-pénème-3-carboxylates d'allyle d'où l'on élimine les groupes protecteurs sur le groupe 1'-hydroxy et sur le groupe 3-carboxyle et d'où l'on isole l'acide (5R,6S,1'R)-2-aminométhyl-6-(1'-hydroxyéthyl)-2-pénème-3-carboxylique.

8. Procédé selon revendication 1, caractérisé en ce que, dans les composés de départ et produits intermédiaires, $R_2^o$ représente un groupe cinnamyloxycarbonyle.

9. Procédé selon revendication 1, caractérisé en ce que l'on fait réagir la (3R,4R,1'R)-3-(1'-hydroxyéthyl)-4-benzoyloxy-2-azétidinone, la (3R,4R,1'R)-3-(1'-hydroxyéthyl)-4-acétoxy-2-azétidinone ou la (3S,4R,1'R)-3-(1'-hydroxyéthyl)-4-tert-butylsulfonyl-2-azétidinone avec la N-cinnamyloxycarbonylthioglycine, ce qui donne la (3S,4R,1'R)-3-(1'-hydroxyéthyl)-4-(N-cinnamyloxycarbonylglycylthio)-2-azétidinone qu'on traite en présence de la N-éthyldiisopropylamine par le chlorureester allylique de l'acide oxalique, ce qui donne des (5R,6S,1'R)-2-allyloxycarbonylaminométhyl-6-[1'-(allyloxyoxalyloxy)-éthyl]-2-pénème-3-carboxylates d'allyle d'où l'on élimine les groupes protecteurs sur le groupe 1'-hydroxy et sur le groupe 3-carboxyle et d'où l'on isole l'acide (5R,6S,1'R)-2-aminométhyl-6-(1'-hydroxyéthyl)-2-pénème-3-carboxylique.

**10.** Composés de formule I selon revendication 1, dans lesquels $R_2$ représente un groupe cinnamyloxycarbonylaminométhyle et $R_1$ et $R_3$ ont les significations indiquées ci-dessus.

**11.** Le (5R,6S,1'R)-(1'-hydroxyéthyl)-2-cinnamyloxycarbonylaminométhyl-pénème-3-carboxylate d'allyle selon revendication 10.

**12.** Composés de formule IV selon revendication 1, dans laquelle $R_2$ représente un groupe cinnamyloxycarbonylaminométhyle,et $R_1$ et Z ont les significations indiquées ci-dessus.

**13.** La (3S,4R,1'R)-3-(1'-hydroxyéthyl)-4-(N-cinnamyloxycarbonylglycylthio)-2-azétidinone selon revendication 12.

**14.** Le (3S,4R,1'R)-2-[4-(N-cinnamyloxycarbonylglycylthio)-3-(1'-allyloxyoxalyloxyéthyl)-2-oxo-1-azétidinyl]-2-oxoacétate d'allyle.

**15.** Le (5R,6S,1'R)-2-cinnamyloxycarbonylaminométhyl-6-[1'-(allyloxyoxalyloxy)-éthyl]-2-pénème-3-carboxylate d'allyle.

**Revendications pour l'Etat contractant suivant : AT**

**1.** Procédé de préparation de composés de formule

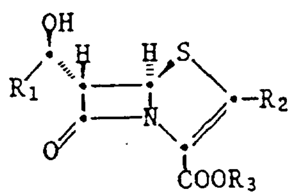

(I),

ou de leurs sels, caractérisé en ce que :

a) on traite un composé de formule

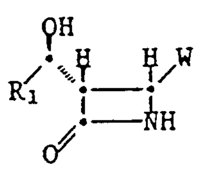

(II)

par un composé introduisant le radical d'acide thiocarboxylique de formule

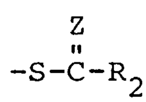

(III)

b) on traite le composé ainsi obtenu, de formule

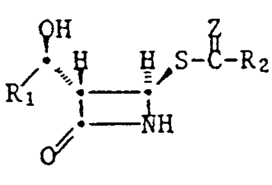

(IV)

par un agent acylant introduisant le radical acyle d'un hémiester oxalique de formule

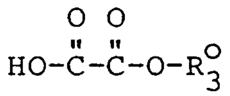 (V)

c) on traite le composé obtenu répondant à la formule

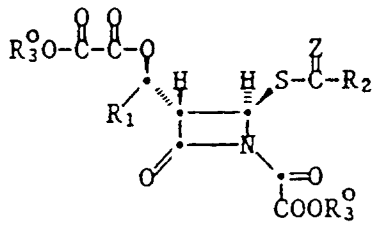 (VII)

par un dérivé organique du phosphore trivalent, et
d) dans le composé obtenu, répondant à la formule

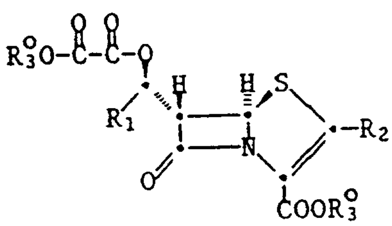 (VIII),

on élimine dans un ordre quelconque le groupe ester oxalique -CO-COO-$R_3^o$ et si on le désire le groupe protecteur $R_3^o$ et/ou un groupe protecteur $R_2^o$ éventuellement présent dans le groupe $R_2$ et on les remplace par l'hydrogène, et si on le désire, on convertit un composé obtenu répondant à la formule I et contenant un groupe salifiable en un sel ou un sel obtenu en le composé libre, les symboles des formules ci-dessus ayant les significations suivantes : $R_1$ représente l'hydrogène ou un groupe alkyle inférieur, $R_2$ représente un radical organique qui peut porter un groupe fonctionnel qui peut éventuellement être protégé par un groupe protecteur usuel $R_2^o$ , $R_3$ représente l'hydrogène ou un groupe protecteur usuel du groupe carboxyle $R_3^o$ , W représente un groupe échangeable contre le radical d'acide thiocarboxylique de formule III et Z représente l'oxygène ou le soufre, $R_1$, $R_2$ et $R_3$ représentant des groupes connus dans la chimie des pénèmes.

**2.** Procédé selon revendication 1, caractérisé en ce que, dans les composés de départ et produits intermédiaires, $R_1$ représente l'hydrogène ou un groupe méthyle, W représente un radical acyloxy, un groupe sulfonyle-$SO_2$-$R_o$ dans lequel $R_o$ représente un radical organique, un radical halogéné ou un groupe azido, Z représente l'oxygène ou le soufre, $R_2$ un radical organique contenant jusqu'à 18 atomes de carbone et qui peut contenir jusqu'à 5 atomes d'azote et/ou jusqu'à 5 atomes d'oxygène et/ou jusqu'à 3 atomes de soufre, et qui peut être relié au cycle pénème aussi bien par l'un de ses atomes de carbone que par un atome d'azote, d'oxygène ou de soufre, et $R_3^o$ représente un groupe protecteur du groupe carboxyle.

**3.** Procédé selon la revendication 1, caractérisé en ce que, dans les composés de départ et produits intermédiaires, $R_1$ représente l'hydrogène ou un groupe méthyle, W représente un groupe alcanoyloxy inférieur, un groupe benzoyloxy ou phényl-alcanoyloxy inférieur éventuellement substitué par des groupes nitro, un groupe -$SO_2$-$R_o$ dans lequel $R_o$ représente un groupe alkyle inférieur éventuellement substitué par un groupe hydroxy, un groupe benzyle ou un groupe phényle éventuellement substitué par exemple par un groupe alkyle inférieur ou un halogène, ou un halogène, Z représente l'oxygène ou le soufre, $R_2$ représente un groupe aliphatyle inférieur, aliphatyloxy inférieur, aliphatylthio inférieur, cycloaliphatyle, cycloaliphatyloxy, cycloaliphatylthio, aryle, aryloxy, arylthio, aryl-aliphatyle inférieur,

aryl-aliphatyloxy inférieur, aryl-aliphatylthio inférieur, hétérocyclyle, hétérocyclyloxy, hétérocyclylthio, hétérocyclyl-aliphatyle inférieur, hétérocyclyl-aliphatyloxy inférieur, hétérocyclyl-aliphatylthio inférieur, hétérocyclyloxy-aliphatyle inférieur ou hétérocyclylthio-aliphatyle inférieur éventuellement substitué, les groupes aliphatyle inférieurs, cycloaliphatyle et hétérocyclyle pouvant être saturés ou insaturés, et $R_3^o$ représente un groupe alkyle inférieur, arylméthyle, hétéroarylméthyle, (alcanoyle inférieur)méthyle, aroylméthyle, halogénoalkyle inférieur, alcényle inférieur, 2-alkylsulfonyle inférieur, 2-cyano-, 2-tri-(alkyle inférieur)-silyl- ou 2-triphénylsilyléthyle.

4. Procédé selon revendication 1, caractérisé en ce que, dans les composés de départ et les produits intermédiaires, $R_3^o$ représente un groupe allyle.

5. Procédé selon revendication 1, caractérisé en ce que, dans les composés de départ et produits intermédiaires, $R_1$ représente l'hydrogène ou un groupe méthyle, W un groupe benzoyloxy, Z l'oxygène ou le soufre, $R_2$ un groupe aminométhyle, 2-amino-propa-1-yle, carbamoyloxyméthyle, 1-méthyltétrazole-5-ylthiométhyle, 2-(1-tétrazolyl)-propyle, 2-oxo-5-tétrahydrofurylméthyle, 2-oxo-5-pyrrolidylméthyle, 2-aminoéthylthio, 2-formamidinoéthylthio, 2-carbamoyloxyéthylthio, 1-imidazolyle, 4,5-diméthyl-1-imidazolyle, 4-méthyl-5-thiazolyle, 5-méthyl-4-thiazolyle et 2-amino-4-méthyl-5-thiazolyle ou leurs dérivés protégés et $R_3^o$ représente un groupe allyle.

6. Procédé selon revendication 1, caractérisé en ce que, dans les composés de départ et produits intermédiaires, $R_2^o$ représente un groupe allyloxycarbonyle.

7. Procédé selon revendication 1, caractérisé en ce que l'on fait réagir la (3A,4R,1'R)-3-(1'-hydroxyéthyl)-4-benzoyloxy-2-azétidinone ou la (3S,4R,1'R)-3-(1'-hydroxyéthyl)-4-tert-butylsulfonyl-2-azétidinone avec la N-allyloxycarbonylthioglycine, ce qui donne la (3S,4R,1'R)-3-(1'-hydroxyéthyl)-4-(N-allyloxycarbonylthioglycylthio)-2-azétidinone qu'on traite en présence de la N-éthyldiisopropylamine par le chlorure-ester allylique de l'acide oxalique, ce qui donne des (5R,6S, 1'R)-2-allyloxycarbonylaminométhyl-6-[1'-(allyloxyoxalyloxy-éthyl]-2-pénème-3-carboxylates d'allyle d'où l'on élimine les groupes protecteurs sur le groupe 1'-hydroxy et sur le groupe 3-carboxyle et d'où l'on isole l'acide (5R,6S,1'R)-2-aminométhyl-6-(1'-hydroxyéthyl)-2-pénème-3-carboxylique.

8. Procédé selon revendication 1, caractérisé en ce que, dans les composés de départ et produits intermédiaires, $R_2^o$ représente un groupe cinnamyloxycarbonyle.

9. Procédé selon revendication 1, caractérisé en ce que l'on fait réagir la (3R,4R,1'R)-3-(1'-hydroxyéthyl)-4-benzoyloxy-2-azétidinone, la (3R,4R,1'R)-3-(1'-hydroxyéthyl)-4-acétoxy-2-azétidinone ou la (3S, 4R,1'R)-3-(1'-hydroxyéthyl)-4-tert-butylsulfonyl-2-azétidinone avec la N-cinnamyloxycarbonylthioglycine, ce qui donne la (3S,4R,1'R)-3-(1'-hydroxyéthyl)-4-(N-cinnamyloxycarbonylglycylthio)-2-azétidinone qu'on traite en présence de la N-éthyldiisopropylamine par le chlorure-ester allylique de l'acide oxalique, ce qui donne des (5R,6S,1'R)-2-allyloxycarbonylaminométhyl-6-[1'-(allyloxyoxalyloxy)-éthyl]-2-pénème-3-carboxylates d'allyle d'où l'on élimine les groupes protecteurs sur le groupe 1'-hydroxy et sur le groupe 3-carboxyle et d'où l'on isole l'acide (5R,6S,1'R)-2-aminométhyl-6-(1'-hydroxyéthyl)-2-pénème-3-carboxylique.